(19) Europäisches Patentamt
European Patent Office
Office européen des brevets

(11) EP 3 088 364 B1

(12) **EUROPEAN PATENT SPECIFICATION**

(45) Date of publication and mention
of the grant of the patent:
**29.05.2019 Bulletin 2019/22**

(21) Application number: **14874349.5**

(22) Date of filing: **24.12.2014**

(51) Int Cl.:
*C01G 9/02* (2006.01)      *A61K 8/27* (2006.01)
*A61Q 17/04* (2006.01)      *A61Q 19/00* (2006.01)
*C09K 3/00* (2006.01)

(86) International application number:
**PCT/JP2014/084115**

(87) International publication number:
**WO 2015/098945 (02.07.2015 Gazette 2015/26)**

(54) **CERIUM-OXIDE-COATED ZINC OXIDE PARTICLES, METHOD FOR PRODUCING SAME, ULTRAVIOLET RAY SHIELDING AGENT, AND COSMETIC**

CEROXIDBESCHICHTETE ZINKOXIDPARTIKEL, VERFAHREN ZUR HERSTELLUNG DAVON, ULTRAVIOLETTSTRAHLUNGSABSCHIRMUNGSMITTEL UND KOSMETIKUM

PARTICULES D'OXYDE DE ZINC RECOUVERTES D'OXYDE DE CÉRIUM, PROCÉDÉ POUR LEUR PRÉPARATION, AGENT PROTECTEUR CONTRE LE RAYONNEMENT ULTRAVIOLET ET PRODUIT COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2013 JP 2013265962**

(43) Date of publication of application:
**02.11.2016 Bulletin 2016/44**

(73) Proprietor: **Sakai Chemical Industry Co., Ltd.**
**Sakai-shi, Osaka 590-8502 (JP)**

(72) Inventors:
• **SUEDA, Satoru**
Iwaki-shi
Fukushima 971-8183 (JP)
• **HASHIMOTO, Mitsuo**
Iwaki-shi
Fukushima 971-8183 (JP)
• **WADA, Mizuho**
Sakai-shi
Osaka 590-0985 (JP)

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(56) References cited:
EP-A2- 1 798 263      WO-A1-2013/035545
WO-A1-2013/042596      JP-A- 2007 161 578
JP-A- 2010 185 133      US-A- 5 976 511

## Description

### TECHNICAL FIELD

The present disclosure relates to a cerium oxide-coated zinc oxide particle, a method for producing the same, an ultraviolet shielding agent, and a cosmetic.

### BACKGROUND OF THE DISCLOSURE

An ultraviolet contained in the sunlight is divided into UV-A radiation at 400 to 320 nm, UV-B radiation at 320 to 290 nm, and UV-C radiation at 290 to 100 nm by the wavelength. The UV-A radiation occupies little over than 97 % of the total solar ultraviolet amount reaching on the ground, and transmits through a glass or a cloud and permeates to the dermal segment in the back of the skin to cause photoaging such as wrinkle, and slackening.

Conventionally, a responding to UV-B radiation having a strong effect on sunburn has been valued as UV protection. However, further research of photoaging has been done in recent years, and the responding to UV-A radiation captures consumer attention.

For shielding UV-A radiation efficiently, it is needed to combine a large amount of organic ultraviolet absorption agents and/or inorganic ultraviolet shielding agents in products. On the other hand, the organic ultraviolet absorption agent is recognized as a sufficient safety material, but some specific ultraviolet absorption agents are used in limited amounts. From the above, it is needed to shield sufficiently UV-A radiation by using only the inorganic ultraviolet shielding agents.

The inorganic ultraviolet shielding agents such as zinc oxide and titanium oxide to be used in a sunscreen product can reveal the ultraviolet protection performance by the scattering effect of ultraviolet on the powder surface and the effect of absorption the ultraviolet into the powder particle. The scattering effect depends on the reflection factor of the particle and the particle size, and the absorption effect depends on the band gap energy (Eg) of the powder particle. The Eg of zinc oxide is 3.2 eV and electronic excitation thereof is direct transition so that zinc oxide can absorb effectively the light at the wavelength of 388 nm or less corresponding substantially to the Eg value. On the other hand, rutile type titanium oxide to be widely used in cosmetic use has the Eg of 3.0 eV, but the electronic excitation of titanium oxide is indirect transition so that the light at the wavelength of about 320 nm or less being smaller than 413 nm corresponding to original Eg value can be absorbed effectively.

On the other hand, cerium oxide is known as other inorganic ultraviolet shielding agent. The Eg of cerium oxide is 3.1 eV and the wavelength corresponding to the value is 400 nm. Therefore, it is expected to absorb effectively UV-A radiation at the wavelength of 400 nm being the upper limit of UV-A radiation or less. However, the electronic excitation of cerium oxide is indirect transition same as titanium oxide, so that it is anticipated that only ultraviolet at the smaller wavelength than that corresponding to original Eg value is absorbed. On this point, the inventors inspected and confirmed that only cerium oxide cannot exert sufficient ultraviolet shielding performance for UV-A radiation. Further, cerium oxide is an expensive material belonging to rare earth and it is not realistic to use it alone for a general use.

In Patent Document 1, a composite particle comprising zinc oxide of which surface is supported by cerium oxide is disclosed as a composite particle for polishing glass. However, in the method of this document, only the use as the glass polishing agent is disclosed, and it is not examined to improve the ultraviolet shielding performance in cosmetic field and so on.

In Patent Documents 2, 3, and 4, zinc oxide particles containing cerium oxide inside the zinc oxide are disclosed. However, it is needed to do a heat treatment at 350°C or more such that the particle shape and size of cerium oxide are changed for producing the above zinc oxide particles. When such heating treatment is performed, cerium oxide particles are coarsened to impair the function of cerium oxide as fine particle so that a sufficient ultraviolet shielding performance cannot be obtained. Further, the particles disclosed in these documents are composite oxides comprising zinc oxide and cerium oxide. In the case of composite oxides, it is assumed that the ultraviolet absorption property and the light scattering property of zinc oxide is lost corresponding to a blending amount of cerium oxide, that is a decreased amount of zinc oxide particle, and sufficient ultraviolet shielding performance for UV-A radiation and UV-B radiation cannot be achieved.

### PRIOR TECHNICAL DOCUMENTS

### PATENT DOCUMENTS

[Patent Document 1] WO 2013/042596

[Patent Document 2] Japanese Kokai Publication Hei6-345427
[Patent Document 3] Japanese Kokai Publication Hei5-222317
[Patent Document 4] Japanese Kokai Publication Sho62-275182

EP-A-1798263 describes a cerium oxide-coated zinc oxide particle and a preparation method thereof, wherein a burning step is performed at the temperature of 800 to 1200°C. US-A-5976511 describes a process for preparing a cerium oxide-coated barium sulfate particle.

SUMMARY OF INVENTION

PROBLEMS TO BE SOLVED BY THE INVENTION

In view of the problems described above, it is one of the objects of the present disclosure to provide zinc oxide particles having improved ultraviolet shielding performance especially in the region of UV-A radiation at 400 to 320 nm.

MEANS FOR SOLVING OBJECT

The present disclosure relates to a cerium oxide-coated zinc oxide particle comprising a matrix raw zinc oxide particle and a cerium oxide layer formed on the surface of the matrix particle, wherein a cerium oxide amount is 5 to 30 wt% relative to 100 wt% of the zinc oxide, a particle diameter of the cerium oxide-coated zinc oxide particles is 0.01 $\mu$m or more, and a specific surface area of the cerium oxide-coated zinc oxide particle is 5.0 $m^2$/g or more, wherein the full width at half maximum of the $CeO_2$ in the X-ray diffraction within the region of diffraction angle $2\theta=28.6\pm0.3°$ is 0.4 or more.
The cerium oxide-coated zinc oxide particle preferably has a hexagonal plate shape or a hexagonal prism shape.
The present disclosure relates to a method for producing the cerium oxide-coated zinc oxide particle as disclosed above, comprising a step (1) of adding an aqueous solution of cerium salt and an alkaline aqueous solution to a water-based slurry of matrix raw zinc oxide particles at a temperature of 10°C to 90°C while keeping a pH at $9\pm3$, and not comprising a step of heat treatment at 350°C or more.
The method for producing a cerium oxide-coated zinc oxide particle preferably comprises a step (2) of drying the particles obtained in the step (1) at above 100°C and less than 350°C for 1 to 24 hours.
The present disclosure relates to an ultraviolet shielding agent comprising the cerium oxide-coated zinc oxide particle as disclosed above.
The present disclosure relates to a cosmetic comprising the cerium oxide-coated zinc oxide particle as disclosed above.

EFFECTS OF THE INVENTION

The cerium oxide-coated zinc oxide particle of the present disclosure have superior ultraviolet shielding performance to normal zinc oxide particle, and a mixture or a composite oxide of zinc oxide and cerium oxide, and have a superior function for shielding UV-A radiation. Cerium oxide particles have a superior ultraviolet shielding effect for only UV-B radiation. So the cerium oxide-coated zinc oxide particles can obtain a higher ultraviolet shielding performance for UV-A radiation than zinc oxide particles alone, cerium oxide alone, and a mixture or a composite thereof. Therefore, the present disclosure has new technology.

BRIEF DESCRIPTION OF THE DRAWINGS

FIG. 1 is a transmission electron microscope photograph of cerium oxide-coated zinc oxide particles obtained in example 1.
FIG. 2 is an X-ray diffraction spectrum of cerium oxide-coated zinc oxide particles obtained in example 1.
FIG. 3 is a transmission electron microscope photograph of cerium oxide-coated zinc oxide particles obtained in example 2.
FIG. 4 is an X-ray diffraction spectrum of cerium oxide-coated zinc oxide particles obtained in example 2.
FIG. 5 is a transmission electron microscope photograph of cerium oxide-coated zinc oxide particles obtained in example 3.
FIG. 6 is an X-ray diffraction spectrum of cerium oxide-coated zinc oxide particles obtained in example 3.
FIG. 7 is a transmission electron microscope photograph of matrix hexagonal plate-shaped zinc oxide particles in comparative example 1.

FIG. 8 is a transmission electron microscope photograph of matrix hexagonal prism-shaped zinc oxide particles in comparative example 2.

FIG. 9 is a transmission electron microscope photograph of zinc oxide particles in comparative example 3.

FIG. 10 is an X-ray diffraction spectrum of zinc oxide particles in comparative example 3.

FIG. 11 is a transmission electron microscope photograph of cerium oxide particles in comparative example 4.

FIG. 12 is an X-ray diffraction spectrum of cerium oxide particles in comparative example 4.

FIG. 13 is a transmission electron microscope photograph of zinc oxide particles in comparative example 6.

FIG. 14 is an X-ray diffraction spectrum of zinc oxide particles in comparative example 6.

FIG. 15 is a transmission electron microscope photograph of matrix zinc oxide particles in comparative example 7.

FIG. 16 is a transmission electron microscope photograph of zinc oxide particles in comparative example 8.

FIG. 17 is an X-ray diffraction spectrum of zinc oxide particles in comparative example 8.

FIG. 18 is a transmission electron microscope photograph of zinc oxide particles in comparative example 9.

FIG. 19 is an X-ray diffraction spectrum of zinc oxide particles in comparative example 9.

FIG. 20 is a transmission electron microscope photograph of zinc oxide particles in comparative example 10.

FIG. 21 is an X-ray diffraction spectrum of zinc oxide particles in comparative example 10.

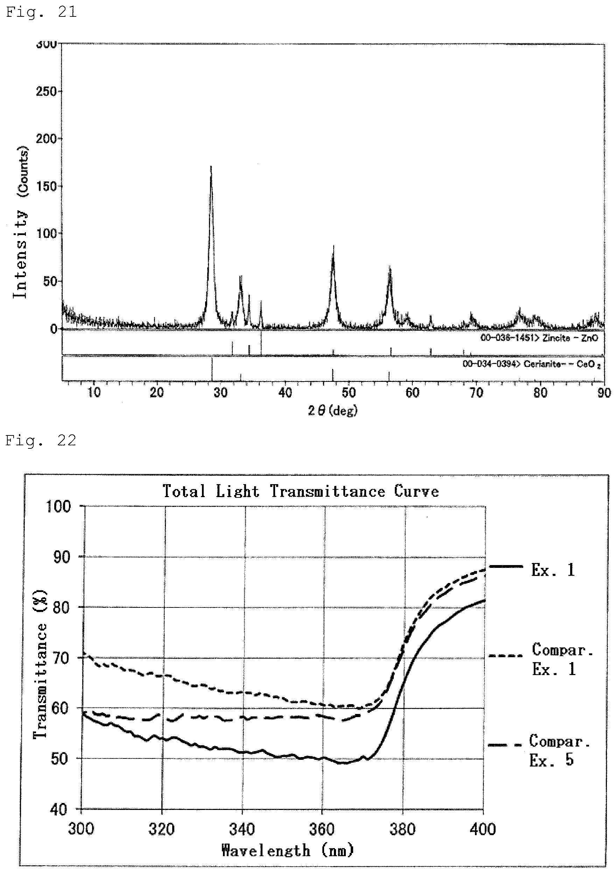

FIG. 22 shows total light transmittance curves in the ultraviolet wavelength region of 300 to 400 nm defined by an ultraviolet shielding ratio of a coating film containing cerium oxide-coated zinc oxide particles of example 1, an ultraviolet shielding ratio of a coating film containing zinc oxide particles of comparative example 1, and an ultraviolet shielding ratio of a coating film of a mixture obtained by mixing zinc oxide particles of comparative example 1 and cerium oxide particles of comparative example 2 in the same ratio as the $ZnO/CeO_2$ composition ratio in the cerium oxide-coated zinc oxide particle of example 1 (comparative example 5).

FIG. 23 is a schematic view illustrating a method for measuring a particle diameter of zinc oxide particles in examples and comparative examples.

Fig. 24 is a schematic view illustrating a method for measuring an aspect ratio of hexagonal plate-shaped zinc oxide particles.

Fig. 25 is a schematic view illustrating a method for measuring an aspect ratio of hexagonal prism-shaped zinc oxide particles.

FIG. 26 is an explanation view of ultraviolet shielding ratio 1 (%) and ultraviolet shielding ratio 2 (%).

## DETAILED DESCRIPTION OF PREFERRED EMBODIMENTS

The present disclosure relates to cerium oxide-coated zinc oxide particles comprising a matrix raw zinc oxide particle and a cerium oxide layer formed on the surface of the matrix particle, and having a particle diameter of 0.01 $\mu$m or more and a specific surface area of 5.0 $m^2/g$ or more, wherein the full width at half maximum of the $CeO_2$ in the X-ray diffraction within the region of diffraction angle $2\theta=28.6 \pm 0.3$ is 0.4 or more.

That is, the inventor found that the ultraviolet shielding ratio at the wavelength of 400 nm or less can be improved by using a zinc oxide particle as a matrix and covering the surface thereof with cerium oxide without impairing the direct transition property of the electronic excitation of zinc oxide. Further, the inventor found that the ultraviolet shielding ratio of the cerium oxide-coated zinc oxide particle containing zinc oxide particle as a matrix is higher than the ultraviolet shielding ratio of a simple mixture or a composite oxide of zinc oxide particle and cerium oxide, and completed the present disclosure.

The cerium oxide-coated zinc oxide particles of the present disclosure are obtained by covering the surface of the zinc oxide particles with cerium oxide. These particles are not composite oxides, and the two kinds of particles exist separately. The cerium oxide particles exist on the surface of the matrix raw zinc oxide particles.

It is not clear the reason why the cerium oxide-coated zinc oxide particles in such condition have an excellent ultraviolet shielding performance, but it is assumed that the ultraviolet absorption property and the light scattering property of the cerium oxide particles covering the surface of the zinc oxide particles can improve an effect of shielding the ultraviolet without impairing the ultraviolet absorption property and the light scattering property of the matrix zinc oxide particles by maintaining the shape and the particle diameter of the raw zinc oxide particles as the matrix of the coated zinc oxide particles.

On the other hand, in a simple mixture and a composite oxide of zinc oxide particles and cerium oxide, it is assumed that only the weight average performance of each particle performance is given, and the ultraviolet absorption property and the light scattering property of zinc oxide equivalent to the mixed amount of cerium oxide being the decreasing amount of zinc oxide particle is lost. When only cerium oxide is prepared by neutralizing precipitation, it is assumed that the obtained cerium oxide particles become aggregates of ultrafine particles so that the sufficient dispersion becomes difficult. Further, it is assumed that the particle aggregation reduce the function of fine particles to decrease the ultraviolet shielding performance.

In this way, it is assumed that the cerium oxide-coated zinc oxide particle has superior performance to a simple mixture of zinc oxide and cerium oxide or an ultraviolet shielding agent composed of zinc oxide and cerium oxide existing inside the zinc oxide.

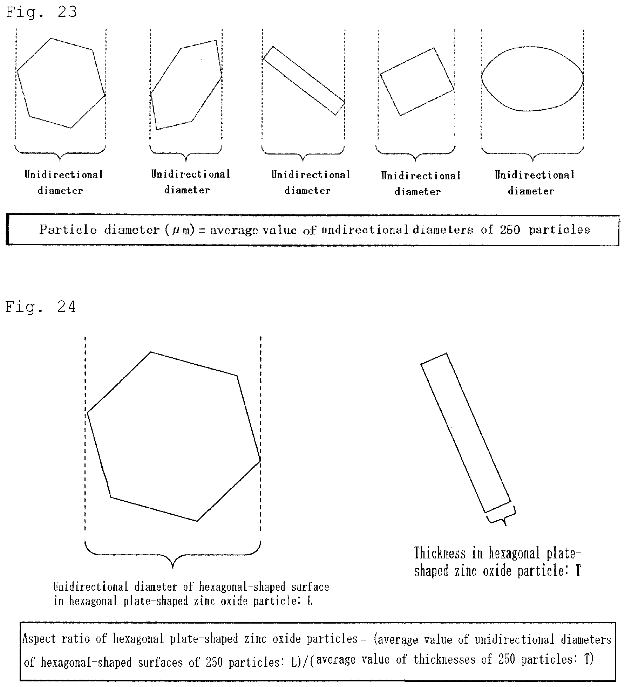

The particle diameter of the cerium oxide-coated zinc oxide particle is 0.01 $\mu$m or more. The cerium oxide-coated zinc oxide particle having the particle diameter within the above-mentioned range is preferred because the ultraviolet shielding performance and physical property of the particles can be expressed sufficiently when contained in a cosmetic and so on. The particle diameter is more preferably 0.02 $\mu$m or more, and still more preferably 0.05 $\mu$m or more. The upper limit of the particle diameter is not particularly limited, but it is more preferably 20 $\mu$m or less, and still more preferably 10 $\mu$m or less. In the specification, the particle diameter of particles is a particle diameter ($\mu$m) defined by a unidirectional diameter in a visual field of 2000 to 50000 magnification in a transmission electron microscope JEM-2100 (manufactured by JEOL Ltd.) photograph (distance between two parallel lines in a fixed direction with a particle held therebetween; measurements are made in a fixed direction regardless of shapes of particles on the image), and is obtained by measuring the unidirectional diameters of 250 particles in the TEM photograph and determining an average value of a cumulative distribution thereof. FIG. 23 is attached for illustrating the measurement method of the particle diameter.

The specific surface area of the cerium oxide-coated zinc oxide particle of the present disclosure is 5.0 m$^2$/g or more, and more preferably 6.0 m$^2$/g or more. When the specific surface area is less than 5.0 m$^2$/g, it is not preferred because cerium oxide particles covering the surface of the zinc oxide particle are coarsened and especially the ultraviolet shielding performance in UV-A radiation region becomes insufficient. The specific surface area (m$^2$/g) is determined by making a measurement using a fully automatic BET specific surface area measuring device Macsorb (manufactured by Mountech Co., Ltd.).

The amount of cerium oxide in the cerium oxide-coated zinc oxide particle/the specific surface area is preferably 2.0 g%/m$^2$ or less. By adjusting the value within the above-mentioned range, a dense cerium oxide layer can be formed on the surface of the zinc oxide particle to improve the ultraviolet shielding performance especially in UV-A radiation region.

That is, when the coated zinc oxide particles are used for a cosmetic, an ink, a coating and so on, an excellent ultraviolet shielding performance can be expressed because the coated zinc oxide particles have superior ultraviolet shielding performance. The ultraviolet shielding performance are values defined by preparing a coating film according to the following method in example and measuring the coating film with the use of a spectrophotometer V-570 (manufactured by JASCO Corporation).

The amount of cerium oxide is 5 to 30 wt% relative to 100 wt% zinc oxide (the matrix zinc oxide particle) in the cerium oxide-coated zinc oxide particle of the present disclosure. It is preferred that the ultraviolet shielding performance can be further improved without impairing the ultraviolet shielding performance of the matrix raw zinc oxide particle of the cerium oxide-coated zinc oxide particle by maintaining the amount of cerium oxide within the above-mentioned range. When the amount of cerium oxide is less than 5 wt%, the ultraviolet shielding performance is insufficient. When the amount of cerium oxide is more than 30wt%, not all the zinc oxide particle is covered by cerium oxide and these compounds are in the mixed state so that the ultraviolet shielding performance is reduced. The amount of cerium oxide is a value determined by measuring Zn amount and Ce amount in terms of oxides using a X-ray fluorescence analyzer ZSX Primus II (manufactured by Rigaku Corporation), and the used software is EZ scan (SQX).

The surface covering of the cerium oxide-coated zinc oxide particle of the present disclosure is preferably dense. Specifically, it means a close formation in which the cerium oxide particles form continuous layers. It is more preferred that there is no void and other trouble in the surface covering layer, and that the covering layer exist as a continuous layer without interruption.

The value of cerium oxide amount/specific surface area in the cerium oxide-coated zinc oxide particle of the present disclosure is preferably 0.01 g%/m$^2$ to 2.0 g%/m$^2$, and more preferably 0.05 g%/m$^2$ to 1.5 g%/m$^2$. The surface of the matrix raw zinc oxide particle can be covered densely and efficiently by CeO$_2$ layer when the value is adjusted within the above-mentioned range. If the value is less than 0.01 g%/m$^2$, the formation becomes a mixture-like state because the difference between the sizes of matrix zinc oxide particle and cerium oxide particle to be used for covering. When the value is over 2.0 g%/m$^2$, the suitable ultraviolet shielding performance cannot be obtained. The value of cerium oxide amount/specific surface area (g%/m$^2$) is a value obtained by dividing the value of X-ray fluorescence analysis value (%) (in terms of CeO$_2$) by the value of the specific surface area (m$^2$/g).

In the cerium oxide-coated zinc oxide particle of the present disclosure, the full width at half maximum of the CeO$_2$ in the X-ray diffraction within the region of diffraction angle 2$\theta$=28.6$\pm$0.3° is 0.4 or more, and preferably 0.5 or more. The surface of the zinc oxide particle can be covered by fine cerium oxide particles without particle coarsening by adjusting the value within the above-mentioned range. When the full width at half maximum of the CeO$_2$ is less than 0.4, the suitable ultraviolet shielding performance cannot be obtained. The full width at half maximum of the CeO$_2$ is measured using an X-ray diffractometer Ultima III (manufactured by Rigaku Corporation) having an X-ray tube with copper.

The cerium oxide composed of the surface covering is preferably particle-shaped in the cerium oxide-coated zinc oxide particle of the present disclosure. The particle diameter of the particle-shaped cerium oxide is preferably 1 to 20 nm. The particle diameter of the cerium oxide is defined by a unidirectional diameter in a visual field of 2000 to 50000 magnification in a transmission electron microscope JEM-2100 (manufactured by JEOL Ltd.) photograph (distance between two parallel lines in a fixed direction with a particle held therebetween; measurements are made in a fixed direction regardless of shapes of particles on the image), and is obtained by measuring the unidirectional diameters of 250 particles in the TEM photograph and determining an average value of a cumulative distribution thereof.

The zinc oxide particles having a particle diameter of 0.01 to 20 $\mu$m are preferably used as the matrix raw zinc oxide particle.

The particle diameter within the above-mentioned range is preferred, because zinc oxide particles having the particle diameter larger than cerium oxide particle can be used as the matrix particle of the cerium oxide-coated zinc oxide particle so that the ultraviolet shielding performance can be improved further while maintaining the properties of the matrix zinc oxide particle. The lower limit is more preferably 0.02 $\mu$m, and still more preferably 0.05 $\mu$m. The upper limit is more preferably 10 $\mu$m, and still more preferably 5 $\mu$m.

Hexagonal plate-shaped zinc oxide particle disclosed in WO 2012/147886 and hexagonal prism-shaped zinc oxide particle disclosed in WO 2012/147887 are preferably used as the matrix raw zinc oxide particle.

When the hexagonal plate-shaped zinc oxide particle and hexagonal prism-shaped zinc oxide particle are used as the matrix raw zinc oxide particle, a superior ultraviolet shielding performance can be shown, and further an excellent function can be expressed as a component for a cosmetic by the physical effect derived from the specific particle shape. Especially, the hexagonal plate-shaped zinc oxide particle have a smooth feeling derived from the hexagonal plate shape, and a superior soft focus property. In the cerium oxide-coated zinc oxide particle of the present disclosure, it is preferred that not only the ultraviolet absorption ability in the region of 400 nm or less can be more improved but also the various functions mentioned above can be provided by containing the hexagonal plate-shaped zinc oxide particle or hexagonal prism-shaped zinc oxide particle having such superior effects as the matrix. These hexagonal plate-shaped zinc oxide particle and hexagonal prism-shaped zinc oxide particle are described below in detail. In addition, methods for producing these particles are disclosed specifically in WO 2012/147886 and WO 2012/147887.

The matrix hexagonal plate-shaped zinc oxide particles preferably have a particle diameter of 0.01 $\mu$m or more. By appropriately controlling the particle diameter of the zinc oxide particles, various kinds of performance such as a proper slippage, a soft focus effect, an ultraviolet shielding property, and a transparency to visible light can be selectively imparted. The particle diameter is more preferably 0.02 $\mu$m or more, and still more preferably 0.03 $\mu$m or more.

The upper limit of the particle diameter is preferably 20 $\mu$m, more preferably 10 $\mu$m, and still more preferably 5 $\mu$m.

The aspect ratio of the matrix hexagonal plate-shaped zinc oxide particle is preferably 2.5 or more. That is, the hexagonal plate-shaped zinc oxide particles are zinc oxide particles having hexagonal plate shape, and particularly when they are used for a cosmetic, good slippage and excellent comfort in use can be achieved owing to the above-mentioned shape. The aspect ratio of hexagonal plate-shaped zinc oxide particle to be used as the matrix is a value determined as a ratio of L/T where L is an average value of measured particle diameters ($\mu$m) of 250 particles, the particle diameter defined by a unidirectional diameter for particles in which the hexagonal-shaped surface of the hexagonal plate-shaped zinc oxide particle faces frontward (distance between two parallel lines in a fixed direction with a particle held therebetween; measurements are made in a fixed direction for particles in which the hexagonal-shaped surface on the image faces frontward), and T is an average value of measured thicknesses ($\mu$m) (length of the shorter side of rectangle) of 250 particles for particles in which the side surface of the hexagonal plate-shaped zinc oxide particle faces frontward (particles that appear rectangular), in a visual field of 2000 to 50000 magnification in a transmission electron microscope JEM-2100 (manufactured by JEOL Ltd.) photograph. For the method for measurement of an aspect ratio, FIG. 24 is attached. The aspect ratio is more preferably 2.7 or more, and still more preferably 3.0 or more.

The particle diameter of the matrix raw zinc oxide particles which is hexagonal prism shaped is preferably 0.1 $\mu$m or more and less than 0.5 $\mu$m. Such Hexagonal prism-shaped zinc oxide particles can achieve both high ultraviolet shielding property and high transparency.

Further, the zinc oxide particles have hexagonal prism shape with an aspect ratio of less than 2.5. That is, the zinc oxide particles are hexagonal prism-shaped zinc oxide particles, and when such hexagonal prism-shaped zinc oxide particles having a small aspect ratio are used especially for a cosmetic, excellent transparency and ultraviolet shielding property can be achieved.

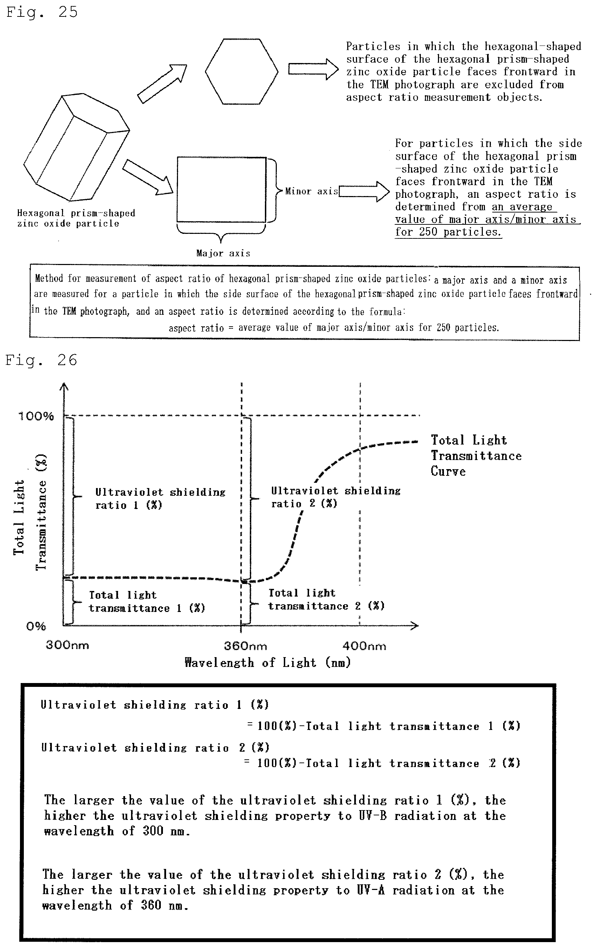

The aspect ratio of the matrix hexagonal prism-shaped zinc oxide particles is determined by the following method. For the aspect ratio of the hexagonal prism-shaped zinc oxide particles, a major axis and a minor axis are measured for particles in which the side surface of the hexagonal prism-shaped zinc oxide particle faces frontward (particles observed as a rectangular or square shape) in a visual field of 2000 to 50000 magnification in a transmission electron microscope JEM-2100 (manufactured by JEOL Ltd.) photograph, and a ratio between the lengths of the major axis and the minor axis: major axis/minor axis is determined. The ratio of major axis/minor axis is measured in the manner described above for 250 hexagonal prism-shaped zinc oxide particles in the TEM photograph, and an average value of a cumulative distribution thereof is determined as an aspect ratio. Hexagonal plate-shaped zinc oxide particles in which the hexagonal-shaped surface faces frontward were excluded from measurement objects because it was difficult to determine the thickness. The method for measurement of an aspect ratio of hexagonal prism-shaped zinc oxide particles is shown in Fig. 25.

A method for producing the cerium oxide-coated zinc oxide particle of the present disclosure is not particularly limited, but the particle can be produced by the following method for producing the cerium oxide-coated zinc oxide particle which falls within the second scope, for example.

The present disclosure relates to a method for producing the cerium oxide-coated zinc oxide particle of the present disclosure. The production method comprises a step of adding an aqueous solution of cerium salt and an alkaline aqueous solution to a water-based slurry of matrix raw zinc oxide particles at a temperature of 10°C to 90°C while keeping a pH at $9\pm3$, and not comprising a step of heat treatment at 350°C or more. The cerium oxide-coated zinc oxide particle having high ultraviolet shielding ratio can be obtained by this production method.

The production method does not comprise a step of heat treatment at 350°C or more.

The cause is unknown, but when the heat treatment at 350°C or more is performed, cerium oxide particles are fusionbonded together and coarsened. Then, by reducing the cerium oxide particle number per unit area on the surface of the cerium oxide-coated zinc oxide particles, the area shielding the ultraviolet in the cerium oxide layer is reduced to degrade the ultraviolet absorption performance. In addition, the light scattering effect in the ultraviolet wavelength region is reduced by coarsening of the cerium oxide particles. Therefore, the cerium oxide-coated zinc oxide particle having a high ultraviolet shielding performance can be obtained without the coarsening of cerium oxide particles by the production method not comprising the step of heat treatment at 350°C or more.

The particle diameter and the particle shape of the matrix raw zinc oxide particles are not particularly limited and the raw zinc oxide particles may have any shapes such as a hexagonal plate shape, a hexagonal prism shape, a spherical shape, a rod shape, a needle shape, a spindle shape or a plate shape. Among them, it is preferred to use the particles having the hexagonal plate shape or the hexagonal prism shape.

The matrix raw zinc oxide particles are added to a liquid medium and prepared a water-based slurry. The liquid medium composing the water-based slurry is preferably water or a mixed liquid of water and a water-soluble organic solvent, and most preferably water. When the mixed liquid of water and a water-soluble organic solvent is used, solvents which can be mixed with water at an arbitrary ratio can be used, for example lower alcohols including methanol and ethanol, acetone, ethylene glycol, diethylene glycol, and polyethylene glycol. The amount of the water-soluble organic solvent to be used is preferably 1 to 30 wt% relative to the total amount of the mixed solvent.

When the slurry is prepared by adding the matrix raw zinc oxide particle to a zinc salt aqueous solution, a zinc oxide concentration is preferably 10 to 500 g/l. And a dispersant may be added according to need.

A method for preparing the slurry is not particularly limited, but a uniform slurry with a zinc oxide concentration of 10 to 500 g/l can be obtained by adding the components to water and dispersing at 10 to 90°C for 5 to 60 minutes, for example.

In the slurry, the pH of the slurry may be adjusted within the prescribed range by adding an acid or a base before reaction initiation to cause a suitable reaction. Specifically, the pH before reaction is preferably 6 or more and less than 13. To do this, it is preferred to add basic compounds such as sodium hydroxide, potassium hydroxide, and ammonia or aqueous solutions thereof.

In the method for producing the coated zinc oxide particle of the present disclosure, an aqueous solution of cerium salt and an alkaline aqueous solution are preferably added simultaneously to the slurry while maintaining the conditions of pH and temperature. By doing this, excellent coated zinc oxide particle can be obtained because a dense cerium oxide layer can be formed on the surface of the zinc oxide particle without dissolving the matrix raw zinc oxide particle. In the case of adding the aqueous solution of cerium salt and the alkaline aqueous solution simultaneously to the slurry, the slurry is preferably stirred. By doing this, a uniform cerium oxide layer can be formed on the surface of the zinc oxide particle. The slurry may be stirred by a usual method, for example, a method using a stirrer and so on.

The cerium salt used in the aqueous solution of cerium salt is not particularly limited but includes cerium salts such as cerium chloride, cerium nitrate, cerium bromide, cerium sulfate, cerium carbonate, and so on.

A concentration of the aqueous solution of the cerium salt is preferably 5 to 60 wt% (in terms of cerium oxide). By using the aqueous solution of the cerium salt with the concentration within the above-mentioned range, a suitable covering may be performed.

An alkaline compound in the alkaline aqueous solution is not particularly limited but includes sodium hydroxide, potassium hydroxide, and ammonia. A concentration of the alkaline aqueous solution is not particularly limited but may be 10 to 500 g/l.

In the production method of the present disclosure, it is preferred to add the aqueous solution of cerium salt and the alkaline aqueous solution with keeping the conditions of pH and temperature. By doing this, the cerium is precipitated uniformly at a constant rate to achieve the purpose suitably.

When the aqueous solution of cerium salt and the alkaline aqueous solution are added, the conditions of pH and temperature are that pH is $9\pm3$ and temperature is 10°C to 90°C. A reaction time is not particularly limited but may be 10 to 360 minutes.

The aqueous solution of cerium salt and the alkaline aqueous solution are preferably added simultaneously onto the different positions of the slurry surface which is the target of the adding. Cerium oxide particles having uniform shape and uniform particle diameter may be deposited to cover the surface of the zinc oxide particles by adding them at the same time. A method for adding is not particularly limited but includes a method of adding a constant amount continuously by a pump. The adding amount is preferably an amount corresponding to the cerium amount in the cerium oxide-coated zinc oxide particle to be desired.

The slurry obtained by the above-mentioned reaction is preferably subjected to a step of filtering, water washing if necessary, and drying the resultant particle at 100°C or more and less than 350°C for 1 to 24 hours. The dying temperature is preferably 100 to 200°C, and still more preferably 100 to 150°C.

By doing this treatment, enough amount of water can be removed while maintaining the particle diameter and the particle shape of the cerium oxide-coated zinc oxide particles . Thereby, a requirement that the specific surface area of the cerium oxide-coated zinc oxide particle is 5.0 $m^2/g$ and more is preferably satisfied. The treatment may be done by usual drying methods using a compartment dryer, a band dryer, a tunnel dryer, a rotary dryer, a flash dryer, or a spray dryer.

In addition, a heat treatment at 350°C or more is not done for producing the cerium oxide-coated zinc oxide particle of the present disclosure. If the heat treatment at 350°C or more is done after the above-mentioned step, a suitable ultraviolet shielding performance cannot be obtained because the cerium oxide particles are coarsened.

The cerium oxide-coated zinc oxide particle of the present disclosure preferably have an ultraviolet shielding ratio higher than that of the matrix raw zinc oxide particle. That is, the cerium oxide covering preferably improve the ultraviolet shielding ratio. Specifically, the ratio, as stated below, of (ultraviolet shielding ratio of coating film containing coated zinc oxide particle (%))/(ultraviolet shielding ratio of coating film containing the raw zinc oxide particle which is the matrix of the coated zinc oxide particle (%)) is preferably 1.1 or more. In the specification, the ultraviolet shielding ratio is a value calculated based on the total light transmittance measured according to the following conditions as to a coating film which is prepared by the method described in example.

(Total light transmittance 1, Total light transmittance 2)

In the specification, the total light transmittance 1 (%) and total light transmittance 2 (%) are values determined by measuring a prepared coating film with the use of a spectrophotometer V-570 (manufactured by JASCO Corporation). The total light transmittance 1 (%) is a value of total light transmittance at the wavelength of 300 nm, and the total light transmittance 2 (%) is a value of total light transmittance at the wavelength of 360 nm. The smaller the total light transmittance 1 (%), the higher the ultraviolet shielding effect to the ultraviolet in UV-B radiation, and the smaller the total light transmittance 2 (%), the higher the ultraviolet shielding effect to the ultraviolet in UV-A radiation.

(Ultraviolet shielding ratio 1, ultraviolet shielding ratio 2)

In the specification, the ultraviolet shielding ratio is calculated according to the following formulas using the above-mentioned total light transmittance.

```
Ultraviolet shielding ratio 1 (%) = 100 % - the total light
transmittance 1 (%)

Ultraviolet shielding ratio 2 (%) = 100 % - the total light
transmittance 2 (%)
```

That is, the value of the ultraviolet shielding ratio 1 (%) means a shielding ratio to the ultraviolet at the wavelength of 300 nm, and the larger this value, the higher the ultraviolet shielding property to UV-B radiation.
The value of the ultraviolet shielding ratio 2 (%) means a shielding ratio to the ultraviolet at the wavelength of 360 nm, and the larger this value, the higher the ultraviolet shielding property to UV-A radiation.
An explanation drawing 26 is attached to make it easy to understand relationships between the total light transmittance 1 (%), the total light transmittance 2 (%), the ultralight shielding ratio 1 (%), and the ultralight shielding ratio 2 (%) .

(The ratio of (ultraviolet shielding ratio 1 of coating film containing the coated zinc oxide particle (%)/(ultraviolet shielding ratio 1 of coating film containing the raw zinc oxide particle which is the matrix of the coated zinc oxide particle (%)))

In the cerium oxide-coated zinc oxide particle of the present disclosure, the ratio of (ultraviolet shielding ratio 1 of a coating film containing the coated zinc oxide particle (%) / (ultraviolet shielding ratio 1 of a coating film containing the raw zinc oxide particle which is the matrix of the coated zinc oxide particle (%)) in UV-B radiation is preferably 1.1 or more.

(The ratio of (ultraviolet shielding ratio 2 of coating film containing the coated zinc oxide particle (%)/(ultraviolet shielding ratio 2 of coating film containing the raw zinc oxide particle which is the matrix of the coated zinc oxide particle (%)))

In the cerium oxide-coated zinc oxide particle of the present disclosure, the ratio of (ultraviolet shielding ratio 2 of a coating film containing the coated zinc oxide particle (%) / (ultraviolet shielding ratio 2 of a coating film containing the raw zinc oxide particle which is the matrix of the coated zinc oxide particle (%))) in UV-A radiation is preferably 1.1 or more.

(Surface treatment)

The cerium oxide-coated zinc oxide particle of the present disclosure may be subjected to a surface treatment. The surface treatment is not particularly limited but includes a surface treatment to form a layer of at least one compound selected from the group consisting of silicon oxides, hydrates of silicon oxide, aluminum oxides, and aluminum hydroxides, a surface treatment using a water-repellent organic compound, and a surface treatment using a coupling agent such as silane coupling agents and titanium coupling agents. These surface treatments may be used in combination.

The formation of a layer using at least one compound selected from the group consisting of silicon oxides, hydrates of silicon oxide, aluminum oxides, and aluminum hydroxides may be done by a method of depositing a Si source compound and/or Al source compound on a powder surface through hydrolysis or thermolysis. The Si source compound and/or Al source compound include compounds which can easily convert to $SiO_2$, $Al(OH)_3$, or $Al_2O_3$ such as tetraalkoxysilane and hydrolysis condensate thereof, sodium silicate, potassium silicate, aluminum alkoxide and hydrolysis condensate thereof, and sodium aluminate.

The hydrolysis reaction is not particularly limited but a method using an acid such as sulfuric acid, hydrochloric acid, acetic acid, and nitric acid may be used. A neutralizing method in the treatment method using the water dispersion may be any one of a method of adding the Si source compound and/or Al source compound after adding the acid to the dispersion, a method of adding the acid after adding the Si source compound and/or Al source compound to the dispersion, and a method of adding the acid and the Si source compound and/or Al source compound at the same time to the dispersion.

The treatment with the water repellent organic compound is not particularly limited but includes a treatment using silicone oils, alkylsilanes, alkyltitanates, alkylaluminates, polyolefins, polyesters, metal soaps, amino acids, or amino acid salts. Among them, silicone oils are preferred because of good chemical stability. The specific example of the silicone oil includes dimethylpolysiloxane (for example, KF-96A-100cs manufactured by Shin-Etsu Chemical Co., Ltd., DM10 manufactured by wacker asahikasei silicone co., ltd.), methyl hydrogen polysiloxane (for example, KF-99P manufactured by Shin-Etsu Chemical Co., Ltd., SH1107C manufactured by Dow corning Toray), (dimethicone/methicone) copolymer (for example, KF-9901 manufactured by Shin-Etsu Chemical Co., Ltd.), methyl phenyl silicone (for example, KF-50-100cs manufactured by Shin-Etsu Chemical Co., Ltd.), amino modified silicone (for example, KF-8015 manufactured by Shin-Etsu Chemical Co., Ltd., JP-8500 Conditioning agent manufactured by Dow corning Toray, ADM6060 manufactured by wacker asahikasei silicone co., ltd.), triethoxysilylethyl polydimethylsiloxyethyl dimethicone (for example, KF-9908 manufactured by Shin-Etsu Chemical Co., Ltd.), and triethoxysilylethyl polydimethylsiloxyethyl hexyl dimethicone (for example, KF-9909 manufactured by Shin-Etsu Chemical Co., Ltd.).

The silane coupling agent includes vinyltris(2-methoxyethoxy)silane, vinyl trichlorosilane, vinyltrimethoxysilane, vinyltriethoxysilane, 2-(3,4 epoxy cyclohexyl)ethyltrimethoxysilane, 3-glycidoxypropyltrimethoxysilane, 3-glycidoxypropylmethyldiethoxysilane, 3-glycidoxypropyltriethoxysilane, p-styryltrimethoxysilane, 3-methacryloxypropyl methyldimethoxysilane, 3-methacryloxypropyl trimethoxysilane, 3-methacryloxypropyl methyldiethoxysilane, 3-methacryloxypropyl triethoxysilane, 3-acryloxypropyl trimethoxysilane, N-2(aminoethyl) 3-aminopropylmethyldimethoxysilane, N-2(aminoethyl) 3-aminopropyltrimethoxysilane, N-2(aminoethyl) 3-aminopropyltriethoxysilane, 3-aminopropyltrimethoxysilane, 3-aminotriethoxysilane, 3-triethoxysilyl-N-(1,3 dimethyl-butylidene) propylamine, N-phenyl-3-aminopropyltrimethoxysilane, N-(vinylbenzyl)-2-aminoethyl-3-aminopropyltrimethoxysilane hydrochloride, 3-ureidopropyltriethoxysilane, 3-chloropropyltrimethoxysilane, 3-mercaptopropylmethyldimethoxysilane, 3-mercaptopropyltrimethoxysilane, bis(triethoxysilylpropyl)tetrasulfide, 3-isocyanatepropyltriethoxysilane, tetramethoxysilane, tetraethoxysilane, methyltrimethoxysilane, methyltriethoxysilane, methyltriethoxysilane, phenyltriethoxysilane, hexamethyldisilazane, hexyltrimethoxysilane, and decyltrimethoxysilane.

The titanium coupling agent includes tetraisopropyl titanate, tetra-n-butyltitanate, butyltitanate dimer, tetra(2-ethylhexyl)titanate, tetramethyl titanate, titanium acetylacetonate, titanium tetraacetylacetonate, titanium ethylacetoacetate, titanium octanedioleate, titanium lactate, titanium triethanolaminato, and polyhydroxy titanium stearate.

The surface treatment is preferably done so that the surface treating amount is 1 to 10 wt% relative to the treated powder as whole. It is preferred to adjust the treating amount within the above-mentioned range because the smoothness and the humidity resistance can be improved to raise the dispersibility in a resin.

The cerium oxide-coated zinc oxide particle of the present disclosure may be used for a cosmetic, an ink, a coating, and a plastic in combination or mixed with other components. The cerium oxide-coated zinc oxide particle especially has the above-mentioned properties so that the cosmetic containing the same which shows an excellent stability and ultraviolet shielding effect can be preferably obtained.

The cosmetic is not particularly limited. Cosmetics for ultraviolet prevention such as a sunscreen agent; cosmetics for base make up such as a foundation; and cosmetics for point make up such as a lipstick can be obtained by mixing the composite powder with any cosmetic raw material, as necessary. When used in cosmetics, excellent performances can be achieved because the composite powders have the ultraviolet shielding performance.

The cosmetic can be in any form, for example, a form of an oil-based cosmetic, a water-based cosmetic, an O/W type cosmetic, or a W/O type cosmetic.

The cosmetic may contain any water-based component or an oil-based component which can be used in the cosmetic field. The water-based component and the oil-based component may contain any component, including, but not limited to, for example, an oil solution, a surfactant, a humectant, a higher alcohol, a sequestering agent, a natural or synthetic polymer, a water-soluble or oil-soluble polymer, an ultraviolet shielding agent, various extracts, a coloring agent such as an organic dye, a preservative, an antioxidant, a colorant, a thickener, a pH adjuster, a perfume, a cooling-sensation agent, an antiperspirant, a bactericidal agent, a skin activating agent, and various powders.

Examples of the oil solution include, but not limited to, for example, natural animal and plant fats (for example, olive oil, mink oil, castor oil, palm oil, beef tallow, evening primrose oil, coconut oil, castor oil, cacao oil, and macadamia nut oil); waxes (for example, jojoba oil, beeswax, lanolin, carnauba wax, and candelilla wax) ; higher alcohols (for example, lauryl alcohol, stearyl alcohol, cetyl alcohol, and oleyl alcohol); higher fatty acids (for example, lauric acid, palmitic acid, stearic acid, oleic acid, behenic acid, and lanolin fatty acid); higher aliphatic hydrocarbons (for example, liquid paraffin, solid paraffin, squalane, vaseline, ceresin, and microcrystalline wax); synthetic ester oils (for example, butyl stearate, hexyl laurate, diisopropyl adipate, diisopropyl sebacate, octyldodecyl myristate, isopropyl myristate, isopropyl palmitate, isopropyl myristate, cetyl isooctanoate, and neopentyl glycol dicaprate); and silicone derivatives (for example, silicone oils such as methyl silicone and methyl phenyl silicone). Further, an oil-soluble vitamin, a preservative, or a whitening agent may be blended.

Examples of the surfactant include a lipophilic nonionic surfactant and a hydrophilic nonionic surfactant. Examples of the lipophilic nonionic surfactant include, but not limited to, for example, sorbitan monooleate, sorbitan monoisostearate, sorbitan monolaurate, sorbitan monopalmitate, sorbitan monostearate, sorbitan sesquioleate, sorbitan trioleate, sorbitan fatty acid esters such as diglycerol sorbitan penta-2-ethylhexylate and diglycerol sorbitan tetra-2-ethylhexylate, glycerin fatty acids such as glycerol mono-cottonseed oil fatty acid, glycerol monoerucate, glycerol sesquioleate, glycerol monostearate, $\alpha,\alpha'$-glycerol oleate pyroglutamate, and glycerol monostearate malate; propylene glycol fatty acid esters such as propylene glycol monostearate; hydrogenated castor oil derivatives; and glycerol alkyl ethers.

Examples of the hydrophilic nonionic surfactant include, but not limited to, for example, POE sorbitan fatty acid esters such as POE sorbitan monooleate, POE sorbitan monostearate, and POE sorbitan tetraoleate; POE sorbit fatty acid esters such as POE sorbit monolaurate, POE sorbit monooleate, POE sorbit pentaoleate, and POE sorbit monostearate; POE glycerin fatty acid esters such as POE glycerin monostearate, POE glycerin monoisostearate, and POE glycerin triisostearate; POE fatty acid esters such as POE monooleate, POE distearate, POE monodioleate, and distearic acid ethylene glycol; POE alkyl ethers such as POE lauryl ether, POE oleyl ether, POE stearyl ether, POE behenyl ether, POE 2-octyl dodecyl ether, and POE cholestanol ether; POE alkyl phenyl ethers such as POE octyl phenyl ether, POE nonyl phenyl ether, and POE dinonyl phenyl ether; Pluaronic types such as Pluronic; POE/POP alkyl ethers such as POE/POP cetyl ether, POE/POP2-decyl tetradecyl ether, POE/POP monobutyl ether, POE/POP hydrogenated lanolin, and POE/POP glycerin ether; tetra POE/tetra POP ethylenediamine condensation products such as Tetronic; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester, and POE hydrogenated castor oil maleic acid; POE beeswax/lanolin derivatives such as POE sorbit beeswax; alkanolamides such as coconut oil fatty acid diethanolamide, lauric acid monoethanolamide, and fatty acid isopropanolamide; POE propylene glycol fatty acid esters, POE alkylamines, POE fatty acid amides, sucrose fatty acid esters, POE nonyl phenyl formaldehyde condensation products, alkyl ethoxydimethylamine oxides, and trioleyl phosphates.

Any other surfactant may be blended, including, for example, anionic surfactants such as fatty acid soaps, higher alkyl sulfate ester salts, POE lauryl sulfate triethanolamine, and alkyl ether sulfate ester salts; cationic surfactants such as alkyl trimethyl ammonium salts, alkyl pyridinium salts, alkyl quaternized ammonium salts, alkyl dimethyl benzylammonium salts, POE alkylamines, alkylamine salts, and polyamine fatty acid derivatives; and amphoteric surfactants such as an imidazoline-based amphoteric surfactant and a betaine-based surfactant, as long as the surfactant does not affect the stability and skin irritation.

Examples of the humectant include, but not limited to, for example, xylitol, sorbitol, maltitol, chondroitin sulfate, hyaluronic acid, mucoitinsulfuric acid, caronic acid, atelocollagen, cholesteryl-12-hydroxystearate, sodium lactate, bile salt, dl-pyrrolidone carboxylate salts, short chain soluble collagen, (EO)PO adducts of diglycerin, Rosa Roxburghii Fruit extract, yarrow extract, and melilot extract.

Examples of the higher alcohol include, but not limited to, for example, linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerin ether (batyl alcohol), 2-decyl tetradecinol, lanolin alcohol, cholesterol, phytosterol, hexyldodecanol, isostearyl alcohol, and octyl dodecanol.

Examples of the sequestering agent include, but not limited to, for example, 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, and edetic acid.

Examples of the natural water-soluble polymer include, but not limited to, for example, plant polymers such as gum arabic, tragacanth gum, galactan, guar gum, carob gum, karaya gum, carrageenan, pectin, agar, quince seed (Cydonia oblonga), algae colloid (brown alga extract), starch (rice, corn, potato, wheat), and glycyrrhizinic acid; microbial polymers such as xanthan gum, dextran, succinoglycan, and pullulan; and animal polymers such as collagen, casein, albumin, and gelatin.

Examples of the semisynthetic water-soluble polymer include, but not limited to, for example, starch polymers such as carboxymethyl starch and methyl hydroxypropyl starch; cellulose polymers such as methylcellulose, nitrocellulose, ethylcellulose, methyl hydroxypropyl cellulose, hydroxyethyl cellulose, cellulose sodium sulfate, hydroxypropyl cellulose, sodium carboxymethyl cellulose (CMC), crystalline cellulose, and cellulose powder; and alginate polymers such as sodium alginate and alginic acid propylene glycol ester.

Examples of the synthetic water-soluble polymer include, but not limited to, for example, vinyl polymers such as polyvinyl alcohol, polyvinyl methyl ether, and polyvinylpyrrolidone; polyoxyethylene polymers such as polyethylene glycol 20,000, 40,000, and 60,000; copolymers such as a polyoxyethylene polyoxypropylene copolymer; acrylic polymers such as sodium polyacrylate, polyethyl acrylate, and polyacrylamide; polyglycerin, polyethylenimine, cationic polymer, carboxyvinyl polymer, alkyl-modified carboxyvinyl polymer, (hydroxyethyl acrylate/acryloyl dimethyl taurine Na)copolymer, (acrylate Na/acryloyl dimethyl taurine Na)copolymer, (acryloyl dimethyl taurine ammonium/ vinylpyrrolidone)copolymer, (acryloyl dimethyl taurine ammonium methacrylate beheneth-25)crosspolymer.

Examples of the inorganic water-soluble polymer include, but not limited to, for example, bentonite, magnesium aluminum silicate (Veegum), laponite, hectorite, and silicic anhydride.

Examples of the ultraviolet shielding agent include, but not limited to, for example, benzoic acid-based ultraviolet shielding agents such as p-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, and N,N-dimethyl PABA butyl ester; anthranilic acid-based ultraviolet shielding agents such as homomenthyl-N-acetyl anthranilate; salicylic acid-based ultraviolet shielding agents such as amyl salicylate, menthyl salicylate, homomenthyl salicylate, octyl salicylate, phenyl salicylate, benzyl salicylate, and p-isopropanol phenyl salicylate; cinnamic acid-based ultraviolet shielding agents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, and glyceryl mono-2-ethylhexanoyl-diparamethoxy cinnamate; benzophenone-based ultraviolet shielding agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methyl benzophenone, 2-hydroxy-4-methoxybenzophenone-5-sulfonate, 4-phenyl benzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor, 3-benzylidene-d,1-camphor, urocanic acid, urocanic acid ethyl ester, 2-phenyl-5-methyl benzoxazole, 2,2'-hydroxy-5-methyl phenyl benzotriazole, 2-(2'-hydroxy-5'-t-octylphenyl)benzotriazole, 2-(2'-hydroxy-5'-methylphenyl benzotriazole, dibenzalazine, dianisoylmethane, 4-methoxy-4'-t-butyldibenzoylmethane, and 5-(3,3-dimethyl-2-norbornylidene)-3-pentan-2-one.

Examples of the other chemical component include, but not limited to, for example, vitamins such as vitamin A oil, retinol, retinol palmitate, inosit, pyridoxine hydrochloride, benzyl nicotinate, nicotinamide, DL-$\alpha$-tocopherol nicotinate, magnesium ascorbyl phosphate, 2-O-$\alpha$-D-glucopyranosyl-L-ascorbic acid, vitamin D2 (ergocalciferol), dl-$\alpha$-tocopherol, DL-$\alpha$-tocopherol acetate, pantothenic acid, and biotin; hormones such as estradiol and ethinyl estradiol; amino acids such as arginine, aspartic acid, cystine, cysteine, methionine, serine, leucine, and triptophan; anti-inflammatory agents such as allantoin and azulene; whitening agents such as arbutin; astringents such as tannic acid; refrigerants such as L-menthol and camphor; sulfur, lysozyme chloride, and pyridoxine chloride.

Examples of various extracts include, but not limited to, for example, Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of various powders include luster color pigments such as red iron oxide, yellow iron oxide, black iron oxide, mica titanium, iron oxide-coated mica titanium, and titanium oxide-coated glass flake; inorganic powders such as mica, talc, kaolin, sericite, titanium dioxide, and silica; and organic powders such as polyethylene powder, nylon powder, crosslinked polystyrene, cellulose powder, and silicone powder. Preferably, some or all of powder components are hydrophobized with a material such as a silicone, a fluorine compound, a metallic soap, an oil solution, or an acyl glutamic acid salt by a known method in order to improve sensory characteristics and makeup retainability. Further, a composite powder other than the composite powder of the present disclosure may be blended and used.

When the cerium oxide-coated zinc oxide particle of the present disclosure is used as a component added to inks, colored pigments such as titanium oxide, red iron oxide, antimony red, cadmium yellow, cobalt blue, Prussian blue, ultramarine, carbon black, and graphite; and extender pigments such as calcium carbonate, kaolin, clay, barium sulfate, aluminum hydroxide, and talc may be used in combination. Further, the above cerium oxide-coated zinc oxide particle can be used with the organic pigment including pigment components such as a soluble azo pigment, an insoluble azo pigment, an azo lake pigment, a condensed azo pigment, a copper phthalocyanine pigment, and a condensed polycyclic pigment; binder resins such as a shellac resin, an acrylic resin, a styrene-acrylic resin, a styrene-maleic acid resin, a styrene-acrylic-maleic acid resin, a polyurethane resin, a polyester resin, and a polyamide resin; and water-miscible organic solvents.

When the cerium oxide-coated zinc oxide particle of the present disclosure is used as a component added to coating compositions, the cerium oxide-coated zinc oxide particle can be used with film-forming resins such as an acrylic resin, a polyester resin, and an epoxy resin; various pigments such as a colored pigment, a extender pigment, and a luster pigment; a curing catalyst, a surface control agent, an antifoaming agent, a pigment dispersant, a plasticizer, a film-forming aid, an ultraviolet absorption agent, an antioxidant, and the like. A resin in the coating may be a curable or uncurable resin.

EXAMPLES

Hereinafter, the present disclosure will be explained with reference to examples. However, the present disclosure is not limited to these examples.

(Example 1)

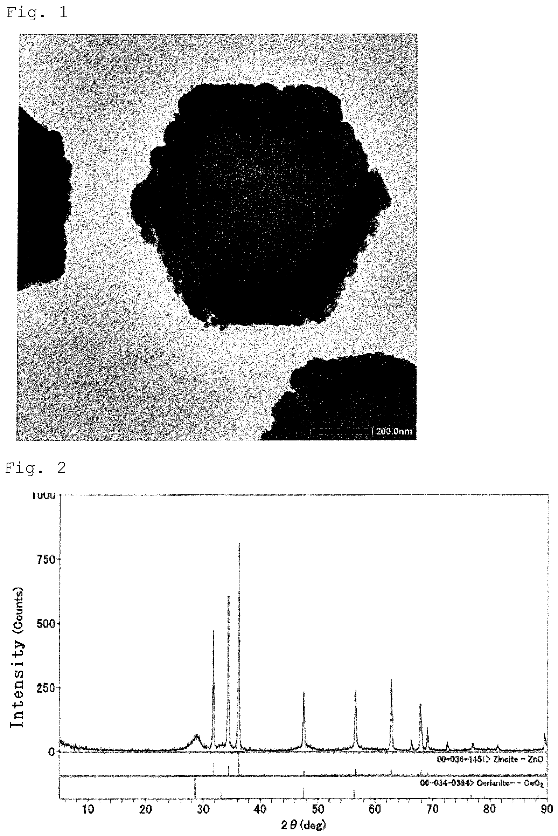

Hexagonal plate-shaped zinc oxide having a particle diameter of 1.05 $\mu$m (XZ-1000F, manufactured by Sakai Chemical Industry Co., Ltd.)150 g was added to water 723.21 g and stirred sufficiently to prepare a water-based slurry with ZnO concentration of 200 g/l. Then, after the slurry was stirred and heated to 45°C, the pH of the slurry was adjusted to 10 by adding 5 wt% of NaOH aqueous solution while maintaining the temperature. Next, 357 ml of cerium nitrate aqueous solution with $CeO_2$ concentration of 42 g/l (corresponding to 10 wt parts relative to the matrix ZnO in terms of $CeO_2$) and 10 wt% of NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours to obtain cerium oxide-coated zinc oxide particles having a particle diameter of 1.09 $\mu$m, which is composed of the matrix hexagonal plate-shaped zinc oxide having a particle diameter of 1.05 $\mu$m and a covering layer of cerium oxide on the surface thereof. The size and form of the obtained particles were observed with a transmission electron microscope JEM-2100 (manufactured by JEOL Ltd.) . The obtained electron microscope photograph is shown in FIG. 1. The obtained particles were analyzed by using an X-ray diffractometer Ultima III (manufactured by Rigaku Corporation). The X-ray diffraction spectra is shown in Fig. 2, and the physical properties of the particle and the coating film are shown in Table 1.

(Example 2)

Hexagonal prism-shaped zinc oxide having a particle diameter of 0.11 $\mu$m (XZ-100P, manufactured by Sakai Chemical Industry Co., Ltd.) 150 g was added to water 723.21 g and stirred sufficiently to prepare a water-based slurry with ZnO concentration of 200 g/l. Then, after the slurry was stirred and heated to 45°C, the pH of the slurry was adjusted to 10 by adding 5 wt% of NaOH aqueous solution while maintaining the temperature. Next, 357 ml of cerium nitrate aqueous solution with $CeO_2$ concentration of 42 g/l (corresponding to 10 wt parts relative to the matrix ZnO in terms of

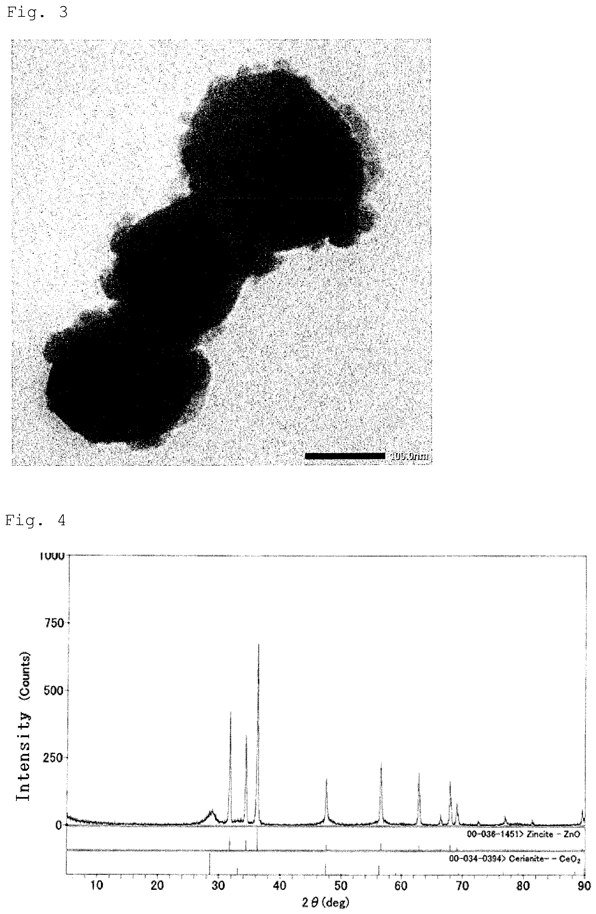

CeO$_2$) and 10 wt% NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours to obtain cerium oxide-coated zinc oxide particles having a particle diameter of 0.12 $\mu$m, which is composed of the matrix hexagonal prism-shaped zinc oxide having a particle diameter of 0.11 $\mu$m and a covering layer of cerium oxide on the surface thereof. The size and form of the obtained particles were observed with a transmission electron microscope JEM-2100 (manufactured by JEOL Ltd.) . The obtained electron microscope photograph is shown in FIG. 3. The obtained particles were analyzed by using an X-ray diffractometer Ultima III (manufactured by Rigaku Corporation). The X-ray diffraction spectra is shown in Fig. 4, and the physical properties of the particle and the coating film are shown in Table 1.

(Example 3)

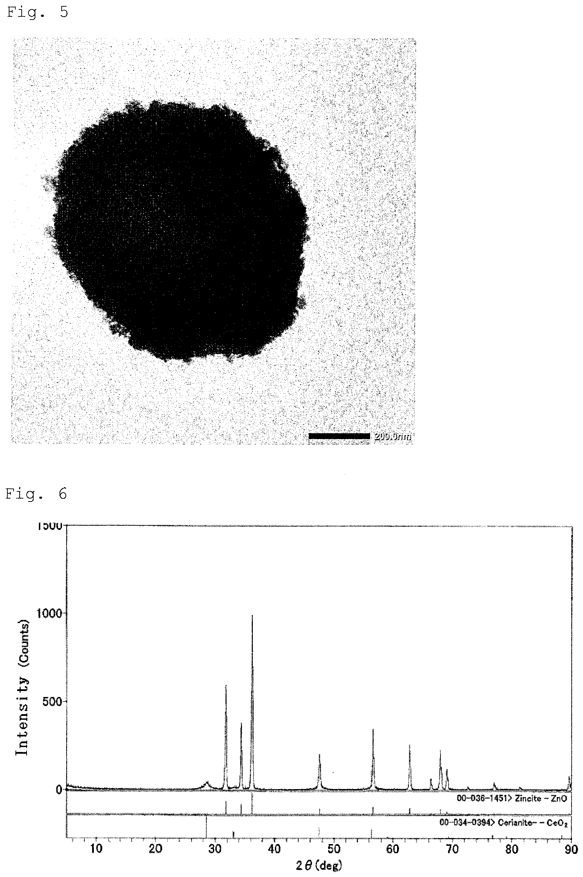

Zinc oxide having a particle diameter of 2.15 $\mu$m (LPZINC-2, manufactured by Sakai Chemical Industry Co., Ltd.) 150 g was added to water 723.21 g and stirred sufficiently to prepare a water-based slurry with ZnO concentration of 200 g/l. Then, after the slurry was stirred and heated to 45°C, the pH of the slurry was adjusted to 10 by adding 5 wt% of NaOH aqueous solution while maintaining the temperature. Next, 357 ml of cerium nitrate aqueous solution with CeO$_2$ concentration of 42 g/l (corresponding to 10 wt parts relative to the matrix ZnO in terms of CeO$_2$) and 10 wt% NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours to obtain cerium oxide-coated zinc oxide particles having a particle diameter of 2.26 $\mu$m, which is composed of the matrix zinc oxide having a particle diameter of 2.15 $\mu$m and a covering layer of cerium oxide on the surface thereof. The size and form of the obtained particles were observed with a transmission electron microscope JEM-2100 (manufactured by JEOL Ltd.). The obtained electron microscope photograph is shown in FIG. 5. The obtained particles were analyzed by using an X-ray diffractometer Ultima III (manufactured by Rigaku Corporation). The X-ray diffraction spectra is shown in Fig. 6, and the physical properties of the particle and the coating film are shown in Table 1.

(Comparative Example 1)

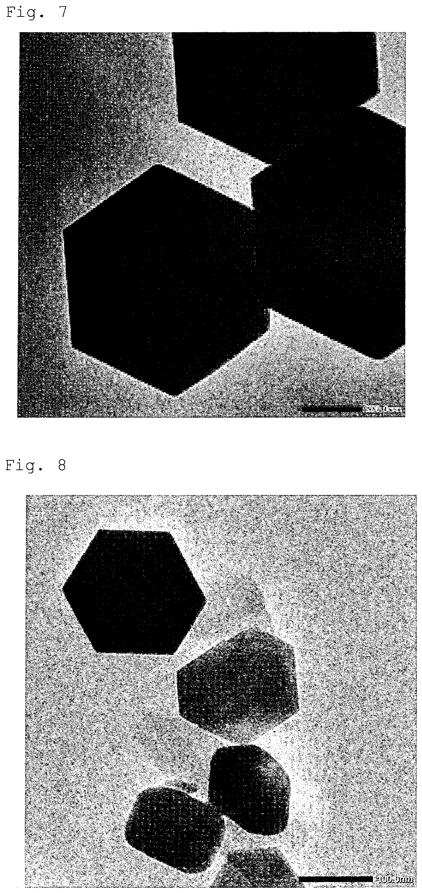

Hexagonal plate-shaped zinc oxide having a particle diameter of 1.05 $\mu$m (XZ-1000F, manufactured by Sakai Chemical Industry Co.) was used as ultraviolet shielding agent for comparison. The obtained transmission electron microscope photograph of particles was shown in FIG. 7. The physical properties of the particle and the coating film are shown in Table 1. Further, this particle is the raw zinc oxide particle to be used as the matrix of coated zinc oxide particles obtained in Example 1, and Comparative example 9.

(Comparative Example 2)

Hexagonal prism-shaped zinc oxide having a particle diameter of 0.11 $\mu$m (XZ-100P, manufactured by Sakai Chemical Industry Co.) was used as ultraviolet shielding agent for comparison. The obtained transmission electron microscope photograph of particles was shown in FIG. 8. The physical properties of the particle and the coating film are shown in Table 1. Further, this particle is the raw zinc oxide particle to be used as the matrix of coated zinc oxide particles obtained in Example 2, and Comparative examples 3 and 6.

(Comparative Example 3)

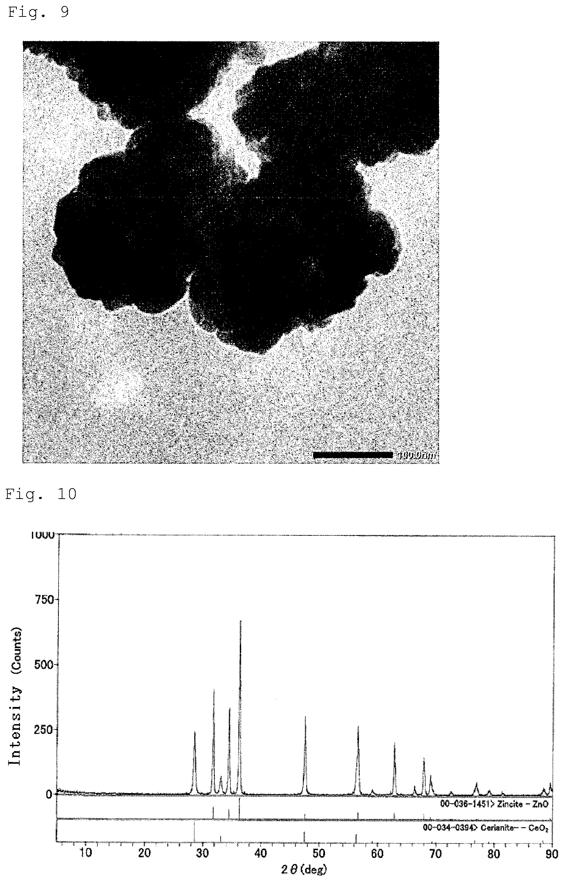

Hexagonal prism-shaped zinc oxide having a particle diameter of 0.11 $\mu$m (XZ-100P, manufactured by Sakai Chemical Industry Co., Ltd.)30 g was added to water 144.64 g and stirred sufficiently to prepare a water-based slurry with ZnO concentration of 200 g/l. Then, after the slurry was stirred and heated to 45°C, the pH of the slurry was adjusted to 10 by adding 5 wt% of NaOH aqueous solution while maintaining the temperature. Next, 143 ml of cerium nitrate aqueous solution with CeO$_2$ concentration of 42 g/l (corresponding to 20 wt parts relative to the matrix ZnO in terms of CeO$_2$) and 5 wt% of NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours. Next, the cerium oxide-coated zinc oxide particles were baked at 1000°C for 2 hours in an electric furnace to obtain cerium oxide-containing zinc oxide particles having a particle diameter of 0.13 $\mu$m composed of zinc oxide particles integrated with cerium oxide particles. The size and form of the obtained particles were observed with a transmission electron microscope JEM-2100 (manufactured by JEOL Ltd.). The obtained electron microscope photograph is shown in FIG. 9. The obtained particles were analyzed by using an X-ray diffractometer Ultima III (manufactured by Rigaku Corporation). The X-ray diffraction spectra is shown in Fig. 10, and the physical properties of the particle and the coating film are shown in Table 1. It was understood that the ultraviolet shielding ratio of the obtained particles is lower than that of the particles obtained in Example 2 having mostly the same particle diameter because the ultraviolet shielding performance depends on the particle diameter.

(Comparative Example 4)

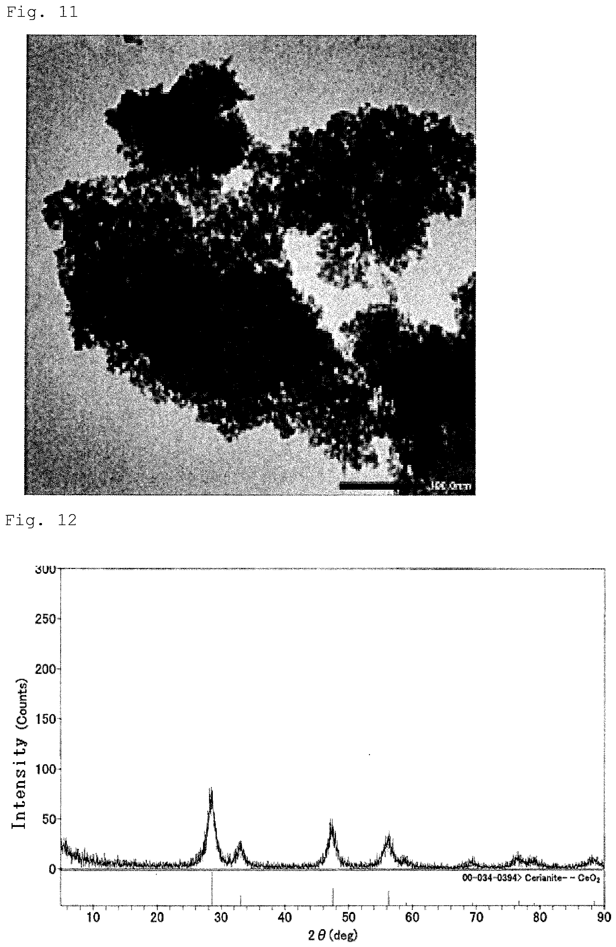

After 750g water was stirred and heated to 45°C, the pH of the slurry was adjusted to 10 by adding 5 wt% of NaOH aqueous solution while maintaining the temperature. Next, 357 ml of cerium nitrate aqueous solution with $CeO_2$ concentration of 42 g/l and 10 wt% of NaOH aqueous solution for neutralizing the cerium nitrate aqueous solution were added simultaneously to the water over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours to obtain fine cerium oxide particles having a particle diameter of 0.007 $\mu$m. The size and form of the obtained particles were observed with a transmission electron microscope JEM-2100 (manufactured by JEOL Ltd.). The obtained electron microscope photograph is shown in FIG. 11. The obtained particles were analyzed by using an X-ray diffractometer Ultima III (manufactured by Rigaku Corporation). The X-ray diffraction spectra is shown in Fig. 12, and the physical properties of the particle and the coating film are shown in Table 1.

(Comparative Example 5)

Hexagonal prism-shaped zinc oxide having a particle diameter of 0.11 $\mu$m of comparative example 2 (XZ-100P, manufactured by Sakai Chemical Industry Co., Ltd.) 9.13 g of and 0.97 g cerium oxide particles having a particle diameter of 0.007 $\mu$m obtained in comparative example 4 were mixed. The obtained mixed powder was used as an ultraviolet shielding agent for comparison. The physical properties of the particles and the coating film are shown in Table 1.

(Comparative Example 6)

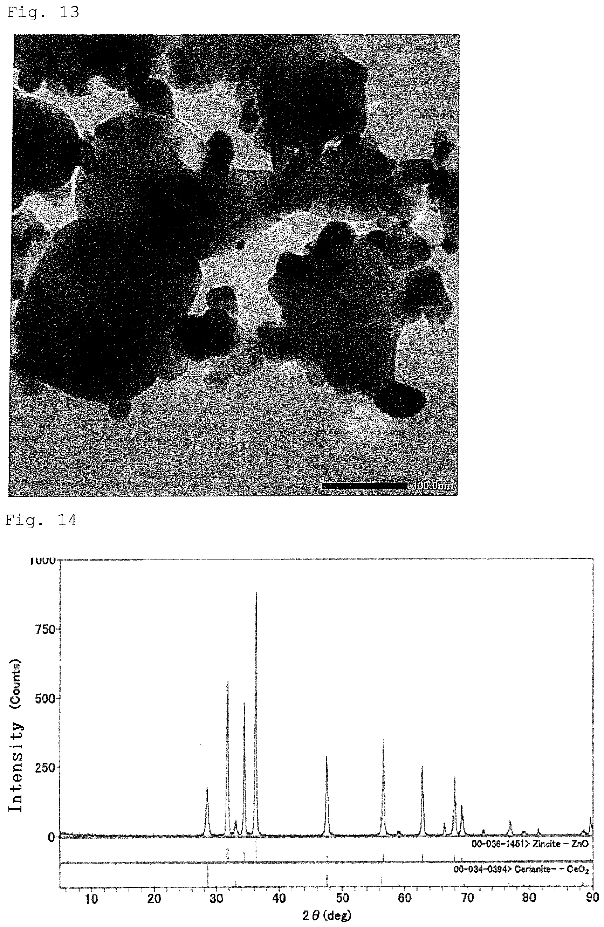

Hexagonal prism-shaped zinc oxide having a particle diameter of 0.11 $\mu$m (XZ-100P, manufactured by Sakai Chemical Industry Co., Ltd.) 150 g was added to water 723.21 g and stirred sufficiently to prepare a water-based slurry with ZnO concentration of 200 g/l. Next, 357 ml of cerium nitrate aqueous solution with $CeO_2$ concentration of 42 g/l (corresponding to 10 wt parts relative to the matrix ZnO in terms of $CeO_2$) and 10 wt% of NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours. Next, the cerium oxide-coated zinc oxide particles were baked at 900°C for 2 hours in an electric furnace to obtain cerium oxide-coated zinc oxide particles having a particle diameter of 0.14 $\mu$m. The particle growth of cerium oxide covering the zinc oxide particle occurred by baking so that the denseness of the surface treating layer was reduced. The size and form of the obtained particles were observed with a transmission electron microscope JEM-2100 (manufactured by JEOL Ltd.). The obtained electron microscope photograph is shown in FIG. 13. The obtained particles were analyzed by using an X-ray diffractometer Ultima III (manufactured by Rigaku Corporation) . The X-ray diffraction spectra is shown in Fig. 14, and the physical properties of the particle and the coating film are shown in Table 1.

(Comparative Example 7)

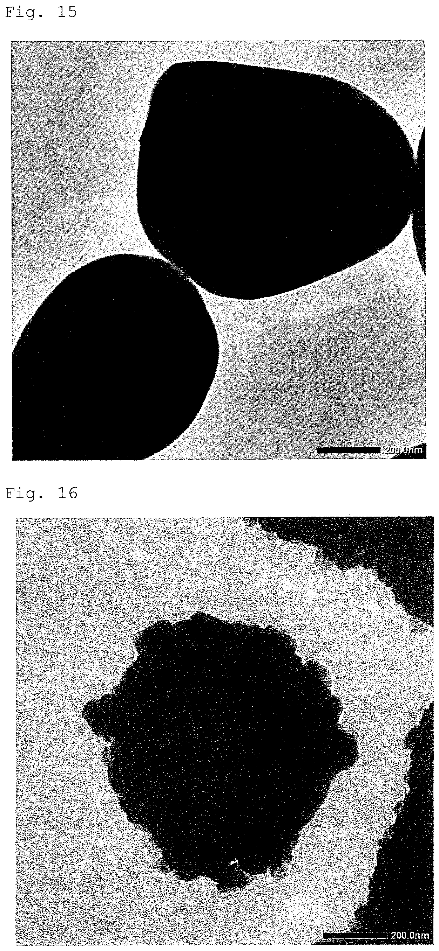

Zinc oxide having a particle diameter of 2.15 $\mu$m (LP-ZINC-2, manufactured by Sakai Chemical Industry Co.) was used as an ultraviolet shielding agent for comparison. The obtained transmission electron microscope photograph of particles was shown in FIG. 15. The physical properties of the particle and the coating film are shown in Table 1. Further, this particle is the raw zinc oxide particle to be used as the matrix of the coated zinc oxide particles obtained in Example 3, and Comparative example 8.

(Comparative Example 8)

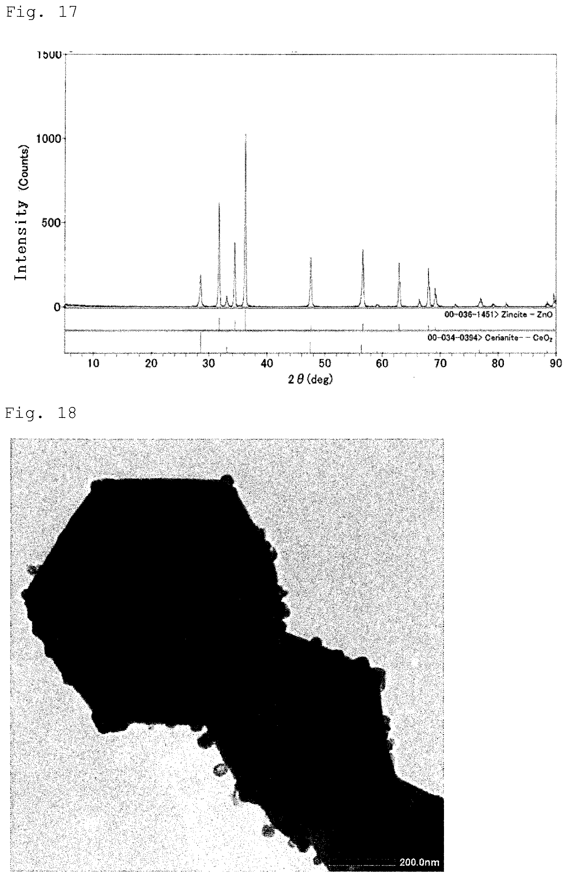

Zinc oxide having a particle diameter of 2.15 $\mu$m (LP-ZINC-2, manufactured by Sakai Chemical Industry Co., Ltd.) 150 g was added to water 723.21 g and stirred sufficiently to prepare a water-based slurry with ZnO concentration of 200 g/l. Then, after the slurry was stirred and heated to 45°C, the pH of the slurry was adjusted to 10 by adding 5 wt% of NaOH aqueous solution while maintaining the temperature. Next, 357 ml of cerium nitrate aqueous solution with $CeO_2$ concentration of 42 g/l (corresponding to 10 wt parts relative to the matrix ZnO in terms of $CeO_2$) and 10 wt% of NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours. Next, the cerium oxide-coated zinc oxide particles were baked at 900°C for 2 hours in an electric furnace to obtain cerium oxide-coated zinc oxide particles having a particle diameter of 2.34 $\mu$m. The particle growth of cerium oxide covering the zinc oxide particle occurred by baking so that the denseness of the surface treating layer was reduced. The size and form of the obtained particles were observed with a transmission electron microscope JEM-2100 (manufactured by JEOL Ltd.). The obtained electron microscope photograph is shown in FIG. 16. The obtained particles were analyzed by using an X-ray diffractometer Ultima III (manufactured by Rigaku Corporation). The X-ray diffraction spectra is shown in Fig. 17, and the physical properties of the particle and the coating film are shown in Table 1. It was understood that the ultraviolet shielding ratio of the obtained particles is lower than that of the particles obtained in Example 3 having mostly the same particle diameter because the ultraviolet shielding performance depends on the particle diameter.

(Comparative Example 9)

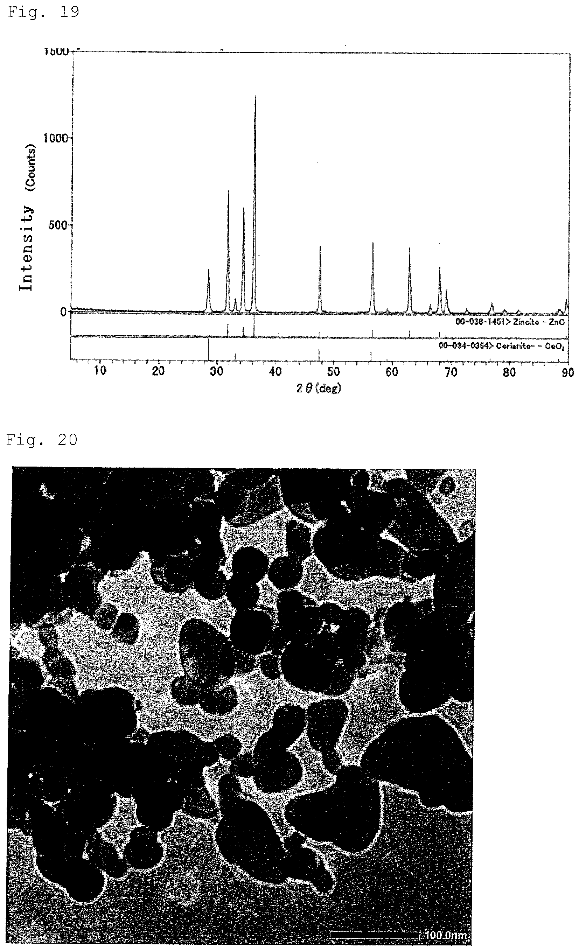

Hexagonal plate-shaped zinc oxide having a particle diameter of 1.05 $\mu$m (XZ-1000F, manufactured by Sakai Chemical Industry Co., Ltd.) 150 g was added to water 723.21 g and stirred sufficiently to prepare a water-based slurry with ZnO concentration of 200 g/l. Then, after the slurry was stirred and heated to 45°C, the pH of the slurry was adjusted to 10 by adding 5 wt% of NaOH aqueous solution while maintaining the temperature. Next, 357 ml of cerium nitrate aqueous solution with $CeO_2$ concentration of 42 g/l (corresponding to 10 wt parts relative to the matrix ZnO in terms of $CeO_2$) and 10 wt% of NaOH aqueous solution for neutralizing the cerium nitrate aqueous solution were added simultaneously to the slurry over 180 minutes while the temperature was maintained at 45°C and the pH was maintained at 10. After the completion of the neutralizing, the mixture was aged for 30 minutes, filtered, and water washed. Then, the mixture was dried at 120°C for 12 hours. Next, the cerium oxide-coated zinc oxide particles were baked at 900°C for 2 hours in an electric furnace to obtain cerium oxide-coated zinc oxide particles having a particle diameter of 1.18 $\mu$m. The particle growth of cerium oxide covering the zinc oxide particle occurred by baking so that the denseness of the surface treating layer was reduced. The size and form of the obtained particles were observed with a transmission electron microscope JEM-2100 (manufactured by JEOL Ltd.). The obtained electron microscope photograph is shown in FIG. 18. The obtained particles were analyzed by using an X-ray diffractometer Ultima III (manufactured by Rigaku Corporation). The X-ray diffraction spectra is shown in Fig. 19, and the physical properties of the particle and the coating film are shown in Table 1. It was understood that the ultraviolet shielding ratio of the obtained particles is lower than that of the particles obtained in Example 1 having mostly the same particle diameter because the ultraviolet shielding performance depends on the particle diameter.

(Comparative Example 10)

Zinc nitrate hexahydrate (manufactured by Kanto Chemical Co., INC., purity of 96%) 70.04 g and cerium nitrate (III) hexahydrate (manufactured by Wako Pure Chemical Industries Ltd., Wako special grade, purity of 98%) 200 g were added to 1200 ml of isobutanol (2-methyl-1-propanol, manufactured by Kishida Chemical Co., Ltd., purity of 99.5% or more) and mixed for 30 minutes with stirring. Then, an aqueous solution of urea 262 g (manufactured by Wako Pure Chemical Industries Ltd., Wako special grade, purity of 99%) and water 1600 g was added to the mixture and stirred. Next, the mixture was hydrolyzed at almost 90°C for almost 4 hours, further at almost 100°C and for 1 hour to obtain a hydrolysis product. This product was filtered and washed by 1000 ml of water followed by drying at 150°C and pulverizing by a hammer mill and a vibrating mill. Then, the product was baked at 600°C for 1 hour to obtain 95.2 g of composite oxide of zinc oxide and cerium oxide having a particle diameter of 0.03 $\mu$m. The size and form of the obtained particles were observed with a transmission electron microscope JEM-2100 (manufactured by JEOL Ltd.) . The obtained electron microscope photograph is shown in FIG. 20. The obtained particles were analyzed by using an X-ray diffractometer Ultima III (manufactured by Rigaku Corporation). The X-ray diffraction spectra is shown in Fig. 21, and the physical properties of the particle and the coating film are shown in Table 1.

【Table 1】

| | | Ex. 1 | Ex. 2 | Ex. 3 | Compar. Ex. 1 | Compar. Ex. 2 | Compar. Ex. 3 | Compar. Ex. 4 | Compar. Ex. 5 | Compar. Ex. 6 | Compar. Ex. 7 | Compar. Ex. 8 | Compar. Ex. 9 | Compar. Ex. 10 |
|---|---|---|---|---|---|---|---|---|---|---|---|---|---|---|
| Physical property of particle | Composition of obtained particle | ZnO+CeO$_2$ | ZnO+CeO$_2$ | ZnO+CeO$_2$ | ZnO | ZnO | ZnO+CeO$_2$ | CeO$_2$ | ZnO+CeO$_2$ (mixture) | ZnO+CeO$_2$ | ZnO | ZnO+CeO$_2$ | ZnO+CeO$_2$ | ZnO+CeO$_2$ |
| | Particle shape | Hexagonal plate shape | Hexagonal prism shape | Indefinite shape | Hexagonal plate shape | Hexagonal prism shape | Indefinite shape | Indefinite shape | | Indefinite shape | Indefinite shape | Indefinite shape | Hexagonal plate shape | Indefinite shape |
| | Particle diameter (μm) | 1.09 | 0.12 | 2.26 | 1.05 | 0.11 | 0.13 | 0.007 | | 0.14 | 2.15 | 2.34 | 1.18 | 0.03 |
| | Aspect ratio | 5.3 | 1.2 | 1.4 | 5.6 | 1.1 | 2.0 | 1.8 | | 1.5 | 1.4 | 1.6 | 4.9 | 1.4 |
| | Fluorescence X-ray analysis value (%) (on ZnO basis) | 90.8 | 90.5 | 89.6 | 100 | 100 | 82.4 | Undetected | 90.9 | 90.5 | 100 | 89.0 | 90.8 | 7.5 |
| | Fluorescence X-ray analysis value (%) (on CeO$_2$ basis) | 9.2 | 9.4 | 9.8 | Undetected | Undetected | 17.4 | 98.8 | 9.3 | 9.4 | Undetected | 10.0 | 9.2 | 92.5 |
| | Specific surface area (m²/g) | 8.5 | 13.2 | 8.7 | 2.0 | 8.7 | 4.4 | 123.2 | | 2.5 | 1.1 | 1.8 | 2.7 | 68.3 |
| | Covering amount of CeO$_2$/Specific surface area (g%/m²) | 1.1 | 0.7 | 1.1 | | | 4.0 | | | 3.8 | | 5.6 | 3.4 | 1.4 |
| | Half peak width of maximum peak of CeO$_2$ | 0.57 | 0.58 | 0.64 | | | 0.36 | 0.78 | | 0.33 | | 0.30 | 0.30 | 0.61 |
| Physical property of coating film | Total light transmittance 1 (%) | 59 | 26 | 65 | 71 | 36 | 45 | 17 | 59 | 44 | 82 | 82 | 70 | 19 |
| | Total light transmittance 2 (%) | 50 | 19 | 65 | 61 | 32 | 44 | 57 | 58 | 41 | 71 | 77 | 65 | 43 |
| | Ultraviolet shielding ratio 1 (%) | 41 | 74 | 35 | 29 | 64 | 55 | 83 | 41 | 56 | 18 | 18 | 30 | 81 |
| | Ultraviolet shielding ratio 2 (%) | 50 | 81 | 36 | 39 | 68 | 56 | 43 | 42 | 59 | 29 | 24 | 35 | 57 |
| | Ratio of (Ultraviolet shielding ratio 1 (%) of coating film containing coated zinc oxide particles)/(Ultraviolet shielding ratio 1 (%) of coating film containing the raw zinc oxide particles as the matrix of the coated zinc oxide particles) | 1.4 | 1.2 | 2.0 | | | 0.9 | | | 0.9 | | 1.0 | 1.0 | |
| | Ratio of (Ultraviolet shielding ratio 2 (%) of coating film containing coated zinc oxide particles)/(Ultraviolet shielding ratio 2 (%) of coating film containing the raw zinc oxide particles as the matrix of the coated zinc oxide particles) | 1.3 | 1.2 | 1.2 | | | 0.8 | | | 0.9 | | 0.8 | 0.9 | |

16

(Evaluation method)

(Composition of obtained Particles)

The X-ray diffraction spectrum shown in Figures 2, 4, 6, 10, 12, 14, 17, 19, and 21 and the compositions of the obtained particles in Table 1 show results of performing analysis using an X-ray diffractometer Ultima III (manufactured by Rigaku Corporation) having an X-ray tube with copper.

(Preparation of Coating Film)

In a mayonnaise bottle having a volume of 75 ml, 2 g of zinc oxide particles in each of examples and comparative examples described above, 10 g of varnish (ACRYDIC A-801-P manufactured by DIC Corporation), 5 g of butyl acetate (special grade reagent, manufactured by Wako Pure Chemical Industries, Ltd.), 5 g of xylene (genuine special grade, manufactured by JUNSEI CHEMICAL CO., LTD.) and 38 g of glass beads (1.5 mm, manufactured by Potters-Ballotini Co., Ltd.) were put and sufficiently mixed, then fixed in a paint conditioner Model 5410 (manufactured by RED DEVIL, Inc.), and subjected to a dispersion treatment by giving vibrations for 90 minutes, thereby preparing a coating. Next, a small amount of the prepared coating was added dropwise onto a slide glass (length/width/thickness = 76 mm/26 mm/0.8 to 1.0 mm, manufactured by Matsunami Glass Ind., Ltd.), and a coating film was prepared using a bar coater (No. 579 ROD No. 6, manufactured by YASUDA SEIKI SEISAKUSHO, LTD.) . The prepared coating film was dried at 20°C for 12 hours, and then used for measurement.

Each coating film was prepared by using the cerium oxide-coated zinc oxide particle of example 1, the hexagonal plate-shaped zinc oxide particle of comparative example 1, and the mixture of hexagonal prism-shaped zinc oxide particles and cerium oxide particles of comparative example 5 based on the above-mentioned composition, and measured by a spectrophotometer V-570 (manufactured by JASCO Corporation). The results of the total light transmittance curves at the ultralight wavelength region of 300 to 400 nm are shown in FIG. 22. The amounts of Zn and Ce contained respectively in the particles of example 1 and in the particle mixture of comparative example 5 are almost the same considering the X-ray fluorescence analysis values in table 1.

From the results of FIG. 22, it is clear that the coated zinc oxide particle of the present disclosure is superior to the matrix raw zinc oxide particle in the ultraviolet shielding ratio for UV-A radiation, and have more excellent ultraviolet shielding performance for UV-A radiation than a simple mixture thereof.

On the other hand, it is clear that the ultraviolet shielding ratio of the coating film containing the cerium oxide-coated zinc oxide particles of comparative example 6 in which the cerium oxide particles in the surface treating layer were coarsened by baking to reduce the denseness of the covering layer and the ultraviolet shielding ratio of coating containing the cerium oxide-containing zinc oxide particles of comparative example 3 in which the zinc oxide particle and the cerium oxide particle were combined by baking are inferior to the ultraviolet shielding ratio of the coating film containing the cerium oxide-coated zinc oxide particle of example 2 composed of the same matrix. Further, it is clear that the cerium oxide-coated zinc oxide particles of example 2 with a dense cerium oxide layer are particles having an excellent ultraviolet shielding performance.

INDUSTRIAL APPLICABILITY

The cerium oxide-coated zinc oxide particle of the present disclosure can be used as a component of a cosmetic, an ink, a coating.

**Claims**

1. A cerium oxide-coated zinc oxide particle comprising a matrix raw zinc oxide particle and a cerium oxide layer formed on the surface of the matrix particle, wherein a cerium oxide amount is 5 to 30 wt% relative to 100 wt% of the zinc oxide, a particle diameter of the cerium oxide-coated zinc oxide particles is 0.01 $\mu$m or more, and a specific surface area of the cerium oxide-coated zinc oxide particle is 5.0 m$^2$/g or more, wherein the full width at half maximum of the CeO$_2$ in the X-ray diffraction within the region of diffraction angle $2\theta$=28.6$\pm$0.3° is 0.4 or more.

2. The cerium oxide-coated zinc oxide particle according to claim 1, having a hexagonal plate shape or a hexagonal prism shape.

3. A method for producing a cerium oxide-coated zinc oxide particle according to claim 1, comprising a step (1) of adding an aqueous solution of cerium salt and an alkaline aqueous solution to a water-based slurry of matrix raw zinc oxide particles at a temperature of 10°C to 90°C while keeping a pH at $9\pm3$, and not comprising a step of heat treatment at 350°C or more.

4. The method for producing a cerium oxide-coated zinc oxide particle according to claim 3, comprising a step (2) of drying the particles obtained in the step (1) at above 100°C and less than 350°C for 1 to 24 hours.

5. An ultraviolet shielding agent comprising the cerium oxide-coated zinc oxide particle according to claim 1 or 2.

6. A cosmetic comprising the cerium oxide-coated zinc oxide particle according to claim 1 or 2.


**Patentansprüche**

1. Mit Ceroxid beschichtetes Zinkoxidteilchen, umfassend ein Matrix-Rohzinkoxidteilchen und eine auf der Oberfläche des Matrixteilchens gebildete Ceroxidschicht, wobei bezogen auf 100 Gew.-% des Zinkoxids eine Ceroxidmenge 5 bis 30 Gew.-% beträgt, ein Partikeldurchmesser der mit Ceroxid beschichteten Zinkoxidteilchen 0.01 $\mu$m oder mehr beträgt, und eine spezifische Oberfläche des mit Ceroxid beschichteten Zinkoxidpartikels 5,0 m$^2$/g oder mehr beträgt, wobei in der Röntgenbeugung innerhalb des Bereichs des Beugungswinkels 2 $\theta$ =28,6 + 0,3° die volle Breite bei halbem Maximum des CeO$_2$ 0,4 oder mehr beträgt.

2. Mit Ceroxid beschichtetes Zinkoxidteilchen nach Anspruch 1 mit einer hexagonalen Plattenform oder einer hexagonalen Prismenform.

3. Verfahren zur Herstellung eines mit Ceroxid beschichteten Zinkoxidteilchens nach Anspruch 1, umfassend einen Schritt (1) der Zugabe einer wässrigen Lösung von Cer-Salz und einer alkalischen wässrigen Lösung zu einer wässrigen Aufschlämmung von Matrix-Rohzinkoxidpartikeln bei einer Temperatur von 10°C bis 90°C, während ein pH-Wert bei $9\pm3$ gehalten wird, und nicht umfassend einen Schritt der Wärmebehandlung bei 350°C oder mehr.

4. Verfahren zur Herstellung eines mit Ceroxid beschichteten Zinkoxidpartikels nach Anspruch 3, umfassend einen Schritt (2) zum Trocknen der in dem Schritt (1) erhaltenen Teilchen bei über 100°C und weniger als 350°C für 1 bis 24 Stunden.

5. Ultraviolettes Abschirmmittel, umfassend das mit Ceroxid beschichtete Zinkoxidteilchen nach Anspruch 1 oder 2.

6. Kosmetikum, umfassend das mit Ceroxid beschichtete Zinkoxidteilchen nach Anspruch 1 oder 2.


**Revendications**

1. Particule d'oxyde de cérium revêtue d'oxyde de zinc comprenant une particule d'oxyde de zinc brut de matrice et une couche d'oxyde de cérium formée sur la surface de la particule de matrice, dans laquelle une quantité d'oxyde de cérium est de 5 à 30% en poids par rapport à 100% en poids de l'oxyde de zinc, un diamètre des particules d'oxyde de zinc revêtu d'oxyde de cérium est 0.01 $\mu$m ou plus, et une surface spécifique de la particule d'oxyde de cérium revêtue d'oxyde de zinc est de 5,0 m$^2$/g ou plus, dans laquelle la largeur totale à la moitié maximum du CeO$_2$ dans la diffraction des rayons X dans la région de l'angle de diffraction 2 =28,6 + 0,3° est 0,4 ou plus.

2. Particule d'oxyde de cérium revêtue d'oxyde de zinc selon la revendication 1, ayant une forme de plaque hexagonale ou de prisme hexagonal.

3. Procédé de production d'une particule d'oxyde de zinc revêtue d'oxyde de cérium selon la revendication 1, comprenant une étape (1) consistant à ajouter une solution aqueuse de sel de cérium et une solution aqueuse alcaline à une suspension aqueuse de particules d'oxyde de zinc brut en matrice à une température de 10°C à 90°C tout en maintenant un pH à $9\pm3$, et ne comprenant pas une étape de traitement thermique à 350°C ou davantage.

4. Procédé de production d'une particule d'oxyde de cérium revêtue d'oxyde de zinc selon la revendication 3, comprenant une étape (2) de séchage des particules obtenues dans l'étape (1) à plus de 100°C et moins de 350°C pendant 1 à 24 heures.

**5.** Agent de protection contre les ultraviolets comprenant la particule d'oxyde de cérium revêtue d'oxyde de zinc selon la revendication 1 ou 2.

**6.** Produit cosmétique comprenant la particule d'oxyde de cérium revêtue d'oxyde de zinc selon la revendication 1 ou 2.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

| Unidirectional diameter | Unidirectional diameter | Unidirectional diameter | Unidirectional diameter | Unidirectional diameter |

Particle diameter ($\mu$m) = average value of undirectional diameters of 250 particles

Fig. 24

Unidirectional diameter of hexagonal-shaped surface in hexagonal plate-shaped zinc oxide particle: L

Thickness in hexagonal plate-shaped zinc oxide particle: T

Aspect ratio of hexagonal plate-shaped zinc oxide particles = (average value of unidirectional diameters of hexagonal-shaped surfaces of 250 particles: L)/(average value of thicknesses of 250 particles: T)

Fig. 25

Particles in which the hexagonal-shaped surface of the hexagonal prism-shaped zinc oxide particle faces frontward in the TEM photograph are excluded from aspect ratio measurement objects.

For particles in which the side surface of the hexagonal prism-shaped zinc oxide particle faces frontward in the TEM photograph, an aspect ratio is determined from an average value of major axis/minor axis for 250 particles.

Hexagonal prism-shaped zinc oxide particle

Minor axis

Major axis

Method for measurement of aspect ratio of hexagonal prism-shaped zinc oxide particles: a major axis and a minor axis are measured for a particle in which the side surface of the hexagonal prism-shaped zinc oxide particle faces frontward in the TEM photograph, and an aspect ratio is determined according to the formula:

aspect ratio = average value of major axis/minor axis for 250 particles.

Fig. 26

Ultraviolet shielding ratio 1 (%)
= 100(%)-Total light transmittance 1 (%)
Ultraviolet shielding ratio 2 (%)
= 100(%)-Total light transmittance 2 (%)

The larger the value of the ultraviolet shielding ratio 1 (%), the higher the ultraviolet shielding property to UV-B radiation at the wavelength of 300 nm.

The larger the value of the ultraviolet shielding ratio 2 (%), the higher the ultraviolet shielding property to UV-A radiation at the wavelength of 360 nm.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013042596 A
- JP HEI6345427 B
- JP HEI5222317 B
- JP SHO62275182 B
- EP 1798263 A
- US 5976511 A
- WO 2012147886 A
- WO 2012147887 A